(19) Europäisches Patentamt / European Patent Office / Office européen des brevets

(11) **EP 2 060 253 A1**

(12) **EUROPEAN PATENT APPLICATION**

(43) Date of publication:
**20.05.2009 Bulletin 2009/21**

(21) Application number: **07380319.9**

(22) Date of filing: **14.11.2007**

(51) Int Cl.:
*A61K 9/16* (2006.01)     *A61K 9/50* (2006.01)
*A61K 31/727* (2006.01)     *A61K 38/28* (2006.01)
*A61K 9/58* (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **LABORATORIOS FARMACEUTICOS ROVI, S.A.**
**28037 Madrid (ES)**

(72) Inventor: **The designation of the inventor has not yet been filed**

(74) Representative: **ABG Patentes, S.L.**
**Avenida de Burgos 16D**
**Edificio Euromor**
**28036 Madrid (ES)**

(54) **Pharmaceutical forms for the release of active compounds**

(57)    Pharmaceutical form containing at least an active compound and a polymeric matrix, wherein said polymeric matrix comprises at least one polymer of cationic nature, process of preparation of said pharmaceutical form, pharmaceutical formulation comprising said pharmaceutical form, and their uses.

**EP 2 060 253 A1**

**Description**

## FIELD OF THE INVENTION

The present invention relates to pharmaceutical forms for the release of active compounds for absorption by mucosal route after their administration, as well as to a process for preparing them, to pharmaceutical formulations containing said pharmaceutical forms and to the use of said pharmaceutical forms and said formulations for the manufacture of a medicament applicable to mucosas.

The route of administration is selected from oral, peroral, sublingual, intraduodenal, intrajejunal, intraileal, intracolonic, rectal, intravaginal, nasal, intrapulmonary, ocular and otic route.

## BACKGROUND OF THE INVENTION

A problem which has been posed in recent years in the pharmaceutical sector is that there is a great variety of active compounds which are characterized fundamentally, in that they cannot be administered by oral route.

The main causes why these compounds cannot be administered by this route are:

- Their rapid enzymatic and metabolic degradation,
- Their chemical and biological instability,
- Their low solubility in aqueous medium or,
- Their limited permeability in the gastrointestinal tract.

Some of the examples of this type of active compounds presenting these problems are:

- Peptidic-type macromolecules such as insulin, interferons or calcitonins,
- Saccharidic-type macromolecules such as heparin or, heparins and derivatives such as LMWH (Low Molecular Weight Heparins), pentasacharides
- Another type of smaller hydrophilic molecules such as salbutamol or acyclovir.

It is necessary to consider that, although some of these compounds can be administered by oral route, they have very low and/or very erratic gastrointestinal absorption, which pose a serious problem for marketing this type of drugs, since the results obtained differ greatly from those expected due to this behaviour in the LADME pharmacokinetic processes (Liberation, Absorption, Distribution, Metabolization and Elimination). Furthermore, these macromolecules have a hydrophilic character and are very poorly permeable, which conditions a low absorption by mucosal routes which, in the case of the oral route, is worsened as these macromolecules have to pass through the stomach.

Like any cellular structure, the digestive mucosa easily allows small lipophilic molecules pass through, but it is very impermeable to charged hydrophilic macromolecules. Furthermore, this aspect is amplified by the fact that the digestive tube is coated with negatively charged mucosa which has a natural tendency to repel the molecules of the same charge.

A clear example of an active compound whose absorption is limited by oral route is heparin, since following its administration by oral route, the absorption through the digestive mucosa will not be limited by its solubility, but by its low permeability with respect to the intestinal membrane, mainly due to its negative charge. The permeability of heparins through the digestive epithelium is, therefore, very low, which makes the administration of heparin/formulations containing heparin by oral route very difficult.

For this reason, the route of administration chosen in the majority of the aforementioned classes of compounds is the route of parenteral administration, mainly via intramuscular or subcutaneous injections. At present, some of these compounds are even administered via nasal and pulmonary formulations as in the case of salmon calcitonin or insulin *[Alpar, H. J.; Somavarapu, S.; Atuah, K. N.; Bramwell, V. W. Adv. Drug Deliv. Rev. 2005, 57, 411-430; Paltz, R. M.; Patton, J. S.; Foster, L.; Mohammed, E. USA Appl. No 355578].*

Nevertheless, a problem posed by these routes of administration is that, in most cases, these treatments require long periods of therapy such as, for example, in some types of diabetes, for which treatment is needed for the entire life, the frequency of administration being daily. This entails a great disadvantage, mainly for the patient, and for this reason it is important to look for alternative routes. Fundamentally, the oral route, that is the most convenient for the patient and the most economical, should correspond to the preferred one. However, for this type of molecules (mainly for oligosaccharides and proteins), designing formulations prepared to be administered by oral route entails a problem and involves many complications, since the gastrointestinal tract degrades these active compounds. This means that they should be formulated to enable, first, the pharmaceutical form to pass through the stomach without degrading the active compound and once it reaches the optimum absorption mucosa, release a large quantity of this active compound selectively on the mucosa wall in a relatively short time interval, consequently, the desired therapeutic or preventive activity is obtained.

In recent decades, numerous vehicles have been developed designed to increase the systemic bioavailability after mucosal administration of numerous traditionally poorly absorbable active compounds, among which we can highlight protein compounds such as insulin *[Norovirus capsid protein expressed in yeast forms virus-like particles and stimulates systemic and mucosal immunity in mice following an oral administration of raw yeast extracts.* Xia et al. Journal of Medical Virology (2006), Volume Date 2007, 79(1), 74-83*; Delivery systems and adjuvants for oral vaccines.* Opinion on Drug Delivery (2006), 3(6), 747-762*; Gastrointestinal absorption of heparin by lipidization or coadministration with penetration enhancers.* Ross et al. Current Drug Delivery (2005), 2(3), 277-287]; antigens *[Gastrointestinal absorption of heparin by lipidization or coadministration with penetration enhancers.* Ross, Benjamin et al. Current Drug Delivery (2005), 2(3), 277-28*; Oral heparin: status review.* Arbi et al. Thrombosis Journal (2006)] and antibodies, as well as polysaccharides such as unfractionated heparins and low molecular weight heparins *[Ximelagatran.* Choudhury et al. Drugs of Today (2006), 42(1), 3-19].

Within the lines of research developed in order to obtain vehicles designed to increase systemic bioavailability after the mucosal administration of numerous active compounds which are traditionally poorly absorbable, the following are included:

- Design of prodrugs *[Prodrug strategies to enhance the intestinal absorption of peptides.* Gangwar et al. Drug Discovery Today (1997), 2(4), 148-155.]
- Use of enzyme metabolism inhibitors [Pharmacokinetic enhancement of protease inhibitor therapy. King et al. Clinical Pharmacokinetics (2004), 43(5), 291-310].
- Development of absorption promoters *[Patent* EP1652836*; patent* IS 200602146].
- Development of mucoadhesive devices such as bioadhesive systems or intestinal patches *[Oral delivery of macromolecules using intestinal patches: applications for insulin delivery.* Journal of Controlled Release (2004), 98(1), 37-45].
- Development of particulate systems

Bioadhesive systems are structures of relatively large size which adhere to the intestinal mucosa after their administration by oral route, significantly increasing the time of intestinal transit of the formulation. Likewise, these devices avoid, to a large extent, the need for the active compound to diffuse through the luminal environment or even through the mucosa coating the absorption epithelium.

An example of these systems is the development of bioadhesive patches, developed for the first time for the systemic absorption of active compounds in the intestine by Eaimtrakarn et al. *[Retention and transit of intestinal mucoadhesive films in the rat small intestine.* Eaimtrakarn et al. International Journal of Pharmaceutics (2001), 224, 61-67] which consist of a four-layer device: (I) a film of coating formed by a water-insoluble polymer which protects the protein active principles from luminal degradation, (II) a surface constituted by a polymer sensitive to the intestinal pH, (III) an intermediate film carrying the active compound and (IV) a bioadhesive film positioned between the intermediate film and the surface coatings designed to generate a high concentration gradient between the patch and the intestine enterocytes.

However, these devices are enormously affected by physiological processes of cell and mucosa turnover in the absorption epithelium. It is for this reason that the use of these devices has not yet managed to avoid the serious problem of variability between administrations, both in relation to the place of adhesion and the contact time of the formulation with the absorption membrane.

To summarize the above, current research in pharmacology (based on particulate systems) is focussed on two different but complementary areas: targeting and controlled release systems.

The release profile of the active compound depends on numerous parameters: size, distribution, porosity, degradation, permeability of the polymer, etc.

The administration of a free drug by oral, intravenous route, etc., normally gives rise to a systemic distribution of the active compound, when what is affected is only a tissue, a local area or a type of cell. From this perspective, it would make much more sense to achieve a targeted action of the drug, especially for those compounds with high toxicity (such as anti-carcinogenic) or for those with a low therapeutic index. For example, administration through these particulate release systems involves an improvement in the administration of the anaesthetic agents, reducing the necessary number of doses, avoiding systemic toxic effects and increasing its concentration in the desired site *[Le Corre, P., Rytting, J. H., Gajan, V., Chevanne, F., Le Verge, R., J. Microencapsulation, 14 (1997) 243 Blanco, M. D., Bernardo, M. V., Gómez, C., Muñiz, E., Teijón, J. M., Biomaterials, 20 (1999) 1919.; Estebe, J. P., Le Corre, P, Chevanne, F., Malljdant, Y., Le Verge, R., Anesth. Analg.81 (1995) 99].

The other channel of research previously mentioned in this field, are controlled release systems. Particulate vectors are formed by polymeric elements which control the release and/or absorption of the active compound through different mechanisms, within which the most typical are the diffusion of the active compound through the pores or channels formed in the polymeric matrix and the degradation/erosion of the polymeric material.

American Patent US 6,475,493 discloses controlled release formulations at acidic pH and quick release in basic media, with aqueous coatings of cores which comprise, in heterogeneous mixture a) at least one water insoluble polymer in a proportion of 75% by weight of the coating; b) an enteric water soluble polymer at pH higher than 6.0 in a proportion of 1-25% by weight of the coating and c) a water-soluble polymer.

The heterogeneous degradation of the polymeric material occurs on the surface of the material which is in contact with the physiological medium. In this case, the rate of degradation is constant and the undegraded material maintains its chemical integrity during the process. Logically, those materials with high surface/volume ratio will degrade faster than the equivalents with a smaller ratio.

Homogeneous degradation involves a random deterioration throughout the polymeric mass. Whilst the molecular weight of the polymer continually decreases, the material can maintain its original shape and retain mass until the polymer has undergone a considerable degradation (even more than 90%), and reaches a critical molecular weight; at that time the solubilization and loss of mass starts [Sáez et al. Liberación Controlada de Fármacos. Revista Iberoamericana de Polímeros. Vol 5(1). 2004].

Patent application US 2005/0020539 A1 discloses pharmaceutical compositions and methods of preparation for the oral administration of heparin for its selective release in the intestine, which comprises a structure of multiple matrices which comprises a) an internal matrix of amphiphilic compounds and lipophilic compounds wherein the active compound is at least partially embedded and b) an outer hydrophilic matrix wherein matrix a) is dispersed.

For the particles to have the desired activity, homogenous degradation should be favoured throughout the polymeric mass to achieve a suitable release, also, a suitable surface potential should be favoured so that the particles approximate the absorption mucosa. Currently the effort of large multinational companies in the pharmaceutical sector is focused on the development of colloidal systems with reduced particle size, as a strategy to increase the systemic bioavailability of active compounds.

It is very well known in scientific literature and in the patent state of the art, how reduced particle size significantly increases the dispersion of the active compound on a large luminal surface, favouring a controlled release of the drug [Potential of poliester microparticles for the sustained release of oral vaccine. Benoit, M. et al. Biopharmaceutics and Pharmaceutical Technology, 1st, Budapest, May 9-11, 1995 (1995), 431-2], decreasing the local concentrations of active compound and enabling high mucoadhesion [Preparation of thiomer microparticles and in vitro evaluation of parameters influencing their mucoadhesive properties. Albrecht, K.; et al. Drug Development and Industrial Pharmacy (2006), 32 (10), 1149-1157], as well as the systemic passage of whole particles [Intestinal absorption of PLGA microspheres in the rat. Damge, C. et al. Journal of Anatomy (1996), 189(3), 491-501] which release the active compound in a controlled manner. Studies carried out by numerous authors reveal the potential shown by these devices for improving intestinal absorption of poorly absorbable molecules, as well as showing the importance of small particle size in intestinal absorption [Transmucosal macromolecular drug delivery. Prego, C. et al. Department Journal of Controlled Release (2005), 101 (1-3), 151-162; Gastrointestinal uptake of biodegradable microparticles: effect of particle size. Desai et al. Pharmaceutical Research (1996), 13(12), 1838-1845].

These colloidal systems include microparticles [Polymeric nano- and microparticle technologies for oral gene delivery. Bhavsar et al. Expert Opinion on Drug Delivery (2007), 4(3), 197-213]; nanoparticles [Lectin-modified solid lipid nanoparticles as carriers for oral administration of insulin. Hang et al. International Journal of Pharmaceutics (2006), 327(1-2), 153-159]; formation of complexes [Stable pharmaceutical formulations comprising macromolecular carriers and methods of use thereof Tan et al. WO2007021970] and liposomes [Investigation of lectin-modified insulin liposomes as carriers for oral administration. Zhang et al. International Journal of Pharmaceutics (2005), 294(1-2), 247-259] among others.

- **Microparticles** are spherical or non-spherical particles, with preferred diameters below 125 $\mu$m. This group includes microcapsules, which are defined as vesicular systems wherein the drug is confined to a cavity surrounded by a single membrane (typically polymeric); and microspheres, which are matricial systems in the form of spherical particles with a size between one and several dozens of microns, without distinction between coating and core, wherein the drug is dissolved or dispersed within a matrix formed by support materials, generally biocompatible polymers and with a large spectrum of release rates and degradative properties [Torrado, J. J., Cadórniga, R., Vectorización, CIF, 8 (1989a) 242]. The drug is released through various mechanisms, among which we can mention surface erosion, the degradation/dissolution of the matrix materials, diffusion, and a combination of diffusion and erosion and erosion or degradation [Torrado, J. J., Cadórniga, R., Farm. Clin., 6 (1989b) 724].

- **Nanoparticles** are submicronic particulate systems (< 1 $\mu$m). Depending on the process used to prepare nanoparticles, nanocapsules or nanospheres can be obtained, these being the morphological equivalents of microcapsules and microspheres, respectively [Rollot, J. M., Couvreur, P., Roblot-Treupel, L., Puisieux, F., J. Pharm. Sci, 75 (1986). 361-364].

The article "Oral bioavailability of a low molecular weight heparin using a polymeric delivery system", Journal of Controlled Release 2006, 113, 38 describes nanoparticles formed by a technique of multiple emulsion and evaporation of solvent, which contains a dispersion of tinzaparin in a polymeric matrix of poly (ε-caprolactone) and Eudragit RS which contains polyvinyl alcohol as surfactant.

The article "Microencapsulation of Low Molecular Weight heparin into Polymeric Particles Designed with Biodegradable and Nonbiodegradable Polycationic Polymers", Drug Delivery 2003, 10, 1 describes microparticles formed by the method of water/oil/water emulsion and evaporation of solvent, which contain low molecular weight heparin, biodegradable polymers such as poly(ε-caprolactone) or poly(D,L-lactic acid-co-glycolic acid) and nonbiodegradable polycationic polymers such as Eudragit® RS or Eduragit® RL) or Eudragit® RS: poly(ε-caprolactone) combinations; Eudragit® RS: poly(D,L-lactic acid-co-glycolic acid); Eudragit® RS: Eudragit® RL: poly(D,L-lactic acid-co-glycolic acid) wherein the polymers are present in equal proportion. Due to the hydrophilic nature of the active substance (low molecular weight heparin (LMWH)) an important diffusion of the active substance is induced through the continuous aqueous phase during the emulsification and solidification procedure. For this reason, the resulting particles have a variable distribution of the active substance through the particle, always having, in a greater or lesser proportion, active substance on the surface. Taking into consideration that they are small-sized particles which have a high specific contact surface with the gastric acids and enzymes, this aspect means that part of the active compound is lost in its passage through the stomach. Furthermore, microparticulate systems, which intrinsically bear a low content of active substance, have a lower encapsulation yield than particles of larger size. Therefore, the amount of active substance they can incorporate is small.

Patent application US 2005/0013866 A1 (whose inventors are co-authors of the article mentioned in the previous paragraph) discloses nanoparticles and microparticles for the oral administration of heparins, peptides and proteins, nucleic acids and growth hormone, formed from a polymeric matrix which comprises at least one biodegradable polymer with at least one polycationic polymer. The biodegradable polymer may be selected from polyesters, poly-ε-caprolactone, polyanhydrides, polyamides, polyurethanes, polyacetals, polyorthoesters and natural polymers and the polycationic polymer may be selected from cellulose derivatives, copolymers of acrylic and methacrylic acid esters such as trimethylammonioethyl methacrylate chloride, chitosan and derivatives and polylysine. The particles are obtained by formation of a water/oil/water emulsion.

The great advantage of these micro- and nanoparticulate systems compared with alternative solutions such as implants is that, due to their small size, they can be injected with a conventional syringe, not requiring surgical intervention. On the other hand, and although it seems paradoxical, it may be easier for a microsphere to be introduced in a cell than for the free drug, since a nanoparticle or microparticle of suitable size is easily incorporated as a vacuole through phagocytosis.

These systems are very interesting as drug carriers which cannot be administered with guarantee by oral route, such as the new protein, peptide, hormone or enzyme drugs which are the product of the biotechnological revolution, and are easily degraded by the gastrointestinal tract enzymes.

Furthermore, polymeric microparticulate systems incorporating anti-carcinogens have been described and clinically tested [Wood, R. W., Li, V. H. K., Kreuter, J., Robinson, J. R., Int. J. Pharm., 23 (1985) 175]; immunosuppressants [Yoshikawa, H., Nakao, Y., Takada, K., Muranishi, S., Wada, R. T., Tabata, Y., Hyon, S. H., Ikada, Y., Chem. Pharm. Bull., 37 (1989) 802.]; vitamins [Sánchez, A., Vila-Jato, J. L., Alonso, M. J., Int. J. Pharm., 99 (1993) 263]; antibiotics, antibacterials agents [Sánchez, A., Vila-Jato, J. L., Alonso, M. J., Int. J. Pharm., 99 (1993) 263J; anti-inflammatories [Dubemet, C., Benoit, J. P., Couarraze, G., Duchène, D., Int. J. Pharm., 35 (1987) 145.] and vaccines [Eldrige, J. H., Staas, J. K., Meulbrock, J. A., McGhee, J. R., Tice, T. R., Gilley, R. M., Mol. Immunol., 28 (1991) 287] with very good results.

Among all of them, nanoparticles (particulate vectors with a diameter below 1 μm) have shown the greatest potential mainly due to the advantages conferred by their reduced size [Nanoencapsulation. II. Biomedical applications and current status of peptide and protein nanoparticulate delivery systems. Reis et al. Nanomedicine (2006), 2(2), 53-65]. These devices may be prepared using polymers such as albumin, ethyl cellulose, gelatine, casein, polyesters, polyanhydrides, polyalkylcyanoacrylates and natural polymers among others [Polymeric nano-and microparticle technologies for oral gene delivery. Bhavsar et al, Expert Opinion on Drug Delivery (2007), 4(3), 197-213; Starch microparticles as vaccine adjuvant. Rydell et al. Expert Opinion on Drug Delivery (2005), 2(5), 807-828] through procedures such as solvent evaporation/extraction [Polymeric nano- and microparticle technologies for oral gene delivery. Bhavsar et al. Expert Opinion on Drug Delivery (2007), 4(3), 197-213]; interfacial polymerization; simple coacervation [Polymeric coacervate microparticles useful for the sustained release administration of therapeutic agents. Heller, Phillip F. WO2006023207; Encapsulation of adenoviral vectors into chitosan-bile salt microparticles for mucosal vaccination. Lameiro et al. Journal of Biotechnology (2006), 126(2), 152-162], complex coacervation [Chitosan: An Atractive biocompatible polymer for macroencasulation..C. Peniche et al. Macromolecules Bioscience, (2003) 3, 511-520; Tramadol release from delivery system based on alginate-chitosan microcapsules. Acosta et al. Macromolecules Bioscience, (2003) 3, 546-551] and precipitation of supercritical fluids [Drug delivery applications of supercritical fluid technology. Sunkara et al. Drug Delivery Technology (2002), 2(1), 44, 46-50], among others.

The advantages conferred by the reduced particle size in the increase of absorption of poorly permeable molecules through the mucosal barrier can be found in numerous publications in the scientific literature [Mucoadhesive nanoparticulate systems for peptide drug delivery. Takeuchi et al. Advanced Drug Delivery Reviews (2001), 47, 39-54; Enteral absorption of insulin in rats from mucoadhesive chitosan-coated liposomes. Takeuchi et al. Pharmaceutical Research (1996), 13, 896-901].

The research carried out by Morishita et al. [Mucosal insulin delivery systems based on complexation polymer hydrogels: effect of particle size on insulin enteral absorption. Morishita et al. Journal of Controlled Release (2004), 97, 115-124] clearly shows the degree in which the reduction in particle size increases systemic bioavailability of insulin when the formulations are administered intestinally. According to published observations by these authors, a reduction in particle size from 180-230 $\mu$m to <43 $\mu$m produces a 18 fold increase in the bioavailability not the insulin administered, going from a systemic bioavailability (relative to the subcutaneous route) of 0.7% to 12.8%.

However, these smaller size particulate systems have a series of drawbacks, for example, for the microspheres, the main obstacle to achieving effective parenteral systems is the degradation and subsequent non-specific elimination by the reticuloendothelial systems, despite the fact that attempts have been made to modify these systems appropriately [Davis, S. S., Illum, L., Colloidal delivery systems-Opportunities and challenges. Site-Specific Drug Delivery, E. Tomlinson, S. S. Davis (Eds), pp.93-110 (1986), John Wiley & Sons Ltd.; UK], this aspect becomes worse in the case of oral route administration, since in this case it has been verified how degradation at stomach acid pH makes the active compound reach the site at which it should be absorbed in very small quantities, causing up to 90% losses in in vitro activity.

For the polymeric conjugate transport systems, their low solubility usually causes problems in their preparation [Duncan, R., Kopekova-Rejmanova, P., Strohalm, J., Hume, I., Cable, H. C., Pohl, J., Lloyd, B., Kopecek, J., Br. J.Cancer, 55 (1987) 165-174.; Endo, N., Umemoto, N., Kato, Y., Takeda, Y., Hara, T., J. Immunol. Methods, 104 (1987) 253-258] and in their injection into the blood stream [Zunino, F., Pratesi, G., Micheloni, A., J. Control. Rel., 10 (1989) 65-73].

Other forms of release of active compounds are micelles which, although they are one of the least studied systems, they base their activity on physicochemical characteristics, especially in organic solvents [Chu, B., Langmuir, 11 (1995) 414-421]. Currently, there are water-soluble micelles related to the chemistry of amphiphilic polymers, which are biocompatible and biodegradable. However, these formulations have limitations with regards to the stability of the active compound to be released.

Homar et al J Microencap 2007, 24:7, 621-633 have studied the influence of polymers on the bioavailability of microencapsulated celecoxib. Microparticles with a size range of 11 - 34 micrometers were prepared using an emulsion method followed by solvent evaporation. Relative bioavailability of celecoxib was below 20% in all cases.

On the other hand, the use of technologies linked to the production of nanoparticles and microparticles is currently limited by a considerable number of factors which condition the subsequent clinical and industrial development thereof.

Among the factors which limit the use of these technologies are:

1. <u>Complex preparation processes.</u>

2. <u>Problematic scaling</u> [Microspheres for controlled release drug delivery. Varde, et al. Expert Opinion on Biological Therapy (2004), 4(1), 35-51].

3. <u>Low yield and encapsulation efficiency</u>: the use of emulsions and/or interfaces in many of the devices based on microparticles and nanoparticles enables the release/migration of the drug substances to be incorporated towards liquids or media which will later be eliminated as part of the preparation process, causing a very high loss of active compound on occasions. In the same way, the preparation processes can generate numerous losses of the matrix-forming material, of the matrix or of the particle coating. The loss of polymeric material in the filters used in the preparation of microparticles by evaporation/solvent extraction and losses by adhesion of matrix-forming material in the preparation of microparticles (o spray drying may serve as an example.

4. <u>Low batch-batch reproducibility</u>. The special sensitivity of colloidal systems to small variations in the particulate vector preparation conditions requires a strict control of all manufacturing variables, paying special attention to environmental factors (temperature, humidity, atmospheric pressure), variations in the excipients or starting materials as well as in the instruments used in their preparation. By way of example, any person skilled in the art may infer that a small increase in the preparation temperature favours the diffusion of the active compound incorporated in the internal phase of a w/o/w (water/oil/water) emulsion towards the external aqueous phase, causing a great loss in active compound content. All these conditioning factors usually cause great batch-to-batch variations, two of the most important aspects affected being:

a) Active compound content, release profile. As just mentioned above, small variations in the preparation conditions or the starting materials may cause large variations in the active compound content of these formulations, enormously hindering the standardization of the process within acceptable limits. Likewise, variations in the active compound content as well as in the particle size of these formulations (fundamentally due to small variations in the stirring conditions and to environmental variations) cause, as have been previously explained, changes in the release of the active compound from these vectors. Thus, an increase in particle size will delay the release of the active compound, modifying its pharmacokinetic profile after its administration and, therefore, its systemic bioavailability.

b) Very broad end product specifications. The batch-to-batch variability, due to the sensitivity of these systems to variations in the preparation conditions, force the establishment of very broad end product specifications in order to validate the production of a batch by a previously standardized industrial procedure. It may serve, for example, to establish specifications for parameters such as active compound content or the release of active compound in a determined time which, as previously indicated, have a large batch to batch variability.

c) Residual solvents: the preparation of particulate systems by procedures such as interfacial polymerization and emulsification, with subsequent evaporation/solvent extraction, often requires the use of very high quantities of organic solvents, for which daily administration is limited based on the majority of the Pharmacopoeias existing at present. Despite the fact that the elimination of these solvents forms part of the preparation procedure of these vectors, the solvent avidity of some polymers typically used, the sensitivity of the particulate vectors to more efficient solvent elimination methods (such as, for example, lyophilization) and the reduced limits for the presence of some of the most commonly used solvents for the preparation of microparticles and nanoparticles (such as, for example, Dichloromethane, classified in the European and United States Pharmacopoeias as Class 2 solvent), may condition the clinical use of microparticles and nanoparticles.

5. Problem of medium/long-term stability [Strategic approaches for overcoming peptide and protein instability within biodegradable nano- and microparticles. Bilati et al. European Journal of Pharmaceutics and Biopharmaceutics (2005), 59(3), 375-388].

6. Particle size-related toxicity. When particulate vectors are administered by mucosal routes, the active compound content therein may be released to the luminal environment by mechanisms already described herein, or the particulate vectors may fully cross the absorption membrane and subsequently release the active compound. In this regard, the particle size constitutes the most important parameter, since a reduction in particle size causes an exponential increase of the material in the form of particles which fully cross the mucosal barrier (Gastrointestinal uptake of biodegradable microparticles: effect of particle size. Desai et al. Pharmaceutical Research (1996), 13(12), 1838-1845]). Unlike larger sized particulate vectors, as may be the case of granules and pellets prepared with polymers, the absorption of the microparticle-forming material and, especially of nanoparticles, forces taking into consideration toxicological aspects related to the delivery of these materials to the systemic circulation. Likewise, any person skilled in the art knows the consequences of the exposure of potentially antigenic materials on the immune response (such as, for example, proteins) in the form of microparticles and nanoparticles on those organs, tissues or bodily compartments to which immunocompetent cells may access, it being possible to generate a wide range of immune responses ranging from the suppression of lymphocyte proliferation to the appearance of hypersensitivity reactions.

7. Restricted access to production technologies and difficulty of access to outsource manufacturing. There are a high number of procedures for the preparation of systems in the form of microparticles and nanoparticles which are currently protected by patents. Likewise, the need to use high solvent quantities, high gas pressures, the establishment of sophisticated control methods of the manufacturing conditions, the high cost of the machinery used and the little presence of these formulations in the market are conditioning factors, known by a person skilled in the art, which raise production costs and condition the existence of facilities which have sufficient manufacturing flexibility to manufacture by contract formulations of this type, also worsened by the complexity in the scaling of the formulations of microparticles and nanoparticles developed in the laboratory.

8. High development and production costs Despite the fact that the results obtained are promising with small-sized particulate systems, in general the systemic bioavailabilities achieved with these types of systems (systems with particle size lower than 100 $\mu$m) are relatively low [Issues in oral nanoparticle drug carrier uptake and targeting. Florence, Alexander T. Journal of Drug Targeting (2004), 12(2), 65-70], the best results being obtained in the administration of molecules with immunological properties wherein the nanoparticles and the microparticles of smaller size have intrinsic activity as antigenic adjuvants [Potential of polymer microencapsulation technology for vaccine innovation. Morris et al. Vaccine (1994), 12(1), 5-11; Particulate systems as adjuvants and carriers for peptide and protein antigens. Liang et al Current Drug Delivery (2006), 3(4), 379-388*].*

All these factors have limited the access of these products to the market and reveal the need to develop vehicles which involve less aggressive manufacturing methods, which have improved stability properties and whose industrial scaling is possible or executable by procedures more commonly used in the pharmaceutical industry.

Despite the fact that the use of granules and pellets has been massive for the oral administration of active compounds, the current state of the art demonstrates that no formulation constituted by them, nor any other derivative thereof such as capsules or tablets, has managed to give rise to significant absorptions of macromolecules whose systemic bioavailability after their non-parenteral administration is more limited by the reduced permeability in the absorption barrier of the molecule than due to the reduced solubility thereof in the luminal fluids.

Patent application US 2005/0281871 A1 discloses a spray coating method for coating granules or pellets wherein the mixture of coating components is performed during the spray process, by simultaneous spray of two aqueous dispersions containing the film forming agents separately, said agents being selected from a) a (meth)acrylate $C_1$-$C_4$ alkyl ester or methacrylic acid in a 30-80% by weight and (meth)acrylate monomers bearing a tertiary amino group in the alkyl radical in 70-20% by weight and b) a polymer bearing anionic groups selected from cellulose derivatives or (meth)acrylate copolymer. Thus avoiding problems related to the formation of dispersed mixtures by spray coating, such as aggregation or coagulation of components and the use of non ionic emulsifiers in 10% by weight and higher, which homogenise the dispersions but that present as drawback an increase in active compound instability.

Therefore, it can be concluded that the latest advances in pharmaceutical technology of oral formulations containing active compounds of the types previously described herein, irrespective of the problems which may be associated to the industrial preparation process, are aimed at increasing the contact surface by adhesives or reducing particle size. These favour contact by increasing their specific surface to achieve significant absorptions of active compounds through the mucosal routes. Particulate systems of greater size are left to one side because the surface potential thereof cannot be calculated (which entails a problem since the surface charge of the particles cannot be calculated to be able to predict the approximation and non-repulsion thereof with the mucosal surface) nor can they be phagocyted by the cells due to greater size and due to the difficulty of those with greater specific surface area to adhere to the mucosa while avoiding repulsion from the very same mucosa.

There remains, therefore, from the information disclosed in the above mentioned documents, the necessity of providing new particulate systems for the administration of active compounds via mucosal route.

Therefore, the object of the present invention is to provide a selective-release pharmaceutical form for administration by mucosal route in high quantities of poorly permeable active compounds, in particular, macromolecular active compounds. The pharmaceutical form can be produced on an industrial scale, resolving the problem of the low homogeneity of batches existing in the case of micro and nanoparticles.

## BRIEF DESCRIPTION OF THE INVENTION

The inventors have previously observed that when the pharmaceutical forms of the prior art were used in greater size, the absorption decreased. One of the purposes of the present invention has been to develop, through different studies, a solid form which had the advantages of the large particle size with regard to scaling and manufacturing, and that also had advantages in the absorption of reduced permeability molecules in the absorption membrane for which the route of oral administration has been traditionally limited.

In general, the conditions aimed to be obtained would be those which would permit that poorly permeable hydrophilic macromolecules, such as those bearing negatively charged groups at pH greater than or equal to 4 were provided with protection against an aggressive luminal environment, and were released at a place and ideal conditions for its absorption.

Following numerous studies, the inventors have designed a model of pharmaceutical form with characteristics which permit the selective release of the active compound in the proximities of the mucosal wall.

The object of the present invention is centred on the use of at least one polymer of cationic nature which forms a matrix in a pharmaceutical form. Said matrix enables achieving significantly high absorptions of molecules whose systemic bioavailability is more limited by the reduced permeability in the absorption barrier due to the reduced solubility thereof in the luminal fluids. Particularly, molecules for which traditionally the route of oral administration has been limited, such as, for example, unfractionated heparins, low molecular weight heparins, fondaparinux and/or proteins such as, for example, insulin.

Therefore, a first aspect of the invention relates to a pharmaceutical form containing at least an active compound and a polymeric matrix, wherein said polymeric matrix comprises at least one polymer of cationic nature.

A second aspect of the invention is a process for preparing the aforementioned pharmaceutical form that comprises combining at least an active compound and at least one polymer of cationic nature in conditions that a polymeric matrix is formed.

In a third aspect, the invention provides a pharmaceutical formulation for the administration of the previously described form.

A forth aspect of the invention is the use of the aforementioned pharmaceutical form for preparing a pharmaceutical formulation.

A fifth aspect of the invention is the use the aforementioned pharmaceutical form or pharmaceutical formulation, for the manufacture of a medicament applicable to a mucosa selected from oropharyngeal mucosa, gastrointestinal mucosa, pulmonary mucosa, nasal mucosa and vaginal mucosa.

A sixth aspect of the invention relates to the use of the afore mentioned pharmaceutical form or pharmaceutical formulation as a medicament applicable to a mucosa selected from the group formed by oropharyngeal mucosa, gastrointestinal mucosa, pulmonary mucosa, nasal mucosa and vaginal mucosa.

Considering the existent prior art discussed herein, it would not be expected that a person skilled in the art, , would be inclined to develop systems of large particle size made through common and easily standardisable processes and achieving significantly high systemic bioavailability of drugs for which the mucosa route has been traditionally inaccessible, improving the release as well as the absorption thereof.

Another purpose of the present invention is to provide a liquid pharmaceutical form sharing some of he advantages of the solid forms. Therefore, a last aspect of the invention is a pharmaceutical form comprising at least at one active principle in combination with a polymer dissolution in a pharmaceutical solvent, wherein the polymer dissolution comprises at least one polymer of cationic nature. This polymer dissolution is intended to harden in contact with gastrointestinal fluids by diffusion of the polymer solvent, forming a polymeric matrix containing the active principle.

## DESCRIPTION OF THE FIGURES

**Figure 1:** depicted is a process for obtaining granulates including a primary granulation step (Diagram 1) and a step of adding the polymers to the primary granules thus formed (Diagram 2).

**Figure 2:** on the left of the figure, dry granules (Gx10) are shown; on the right, granules dispersed in 4% PVA solution (Gx10) are shown.

**Figure 3:** Average manufacturing yields of different granules prepared with 2000 (A), 3000 (B), 4000 (C) and 5000 (D) U anti-Xa of enoxaparin after mixing with Eudragit® RS 30 D (RS), and Aquacoat® ECD (AqC); 100/0, 75/25, 50/50, 25/75, 0/100 % (n=3 $\pm$ standard deviation)

**Figure 4:** Water content of the primary granules and of the granules with polymer mixture by the addition of Eudragit® RS 30 D and Aquacoat® ECD in equivalent quantities (50/50) after 2, 4, 8 and 24 hours of drying at 40°C (n=3 $\pm$ standard deviation).

**Figure 5:** Release kinetics of enoxaparin in % from different primary granules of microcrystalline cellulose mixed with aqueous suspensions containing Eudragit® RS 30 D and/or Aquacoat® ECD prepared with 2000 (A), 3000 (B), 4000 (C) or 5000 (D) U anti-Xa of enoxaparin (n=6 $\pm$ standard deviation).

**Figure 6:** Release kinetics of enoxaparin from primary granules prepared with different quantities of enoxaparin and later mixed with an aqueous suspension containing 100% Eudragit® RS 30 D (n=6 $\pm$ standard deviation).

**Figure 7:** Anti-Xa activity per ml in plasma of New Zealand White rabbits after the oral administration of 600 IU anti-Xa/kg enoxaparin granules in gelatine capsules. The groups correspond to MCC cores mixed respectively with a suspension which contains Aquacoat and Eudragit RS (Aquac+Eudr RS), a suspension of Eudragit RS (Eudr RS) and a suspension of Aquacoat (Aquac) (n=3).

**Figure 8:** Average size of the granules produced by addition of Eudragit RS (left) or by the addition of Eudragit RS and PCL in equivalent quantities (right) (n=3 $\pm$ standard deviation). (1x) corresponds to the quantity added in one stage. (2x) corresponds to the quantity added in two stages of 200 mg of polymers (in the case of Eudragit RS and PCL mixture) or polymer (in the case of the addition of Eudragit RS alone) each. (3x) corresponds to the quantity added in three stages of 200 mg each. (4x) corresponds to the quantity added in four stages of 200 mg each.

**Figure 9:** Release kinetics of tinzaparin from primary granules mixed with different quantities of Eudragit RS dissolved in acetone (n=3). The graph on the left corresponds to the addition of Eudragit in a single stage,

**Figure 10:** Release kinetics of tinzaparin from primary granules mixed with different quantities of Eudragit RS and PCL dissolved in acetone (n=3). The graph on the left corresponds to the addition of Eudragit and PLC in a single stage, whilst the graph on the right corresponds to the addition of the polymer mixture in stages of 200 mg each.

**Figure 11:** Anti-Xa activity per ml in plasma of New Zealand White rabbits after the oral administration of 600 IU anti-Xa/kg of tinzaparin granules in gelatine capsules. Two groups correspond to primary granules of MCC with tinzaparin mixed respectively with 200 and 800 mg of a Eudragit RS and PCL solution in acetone (200mg RS/PCL and 800mg RS/PCL) and the third group corresponds to primary granules of MCC with tinzaparin mixed with 800 mg of Eudragit RS (n=3).

**Figure 12:** Anti-Xa activity per ml in plasma of New Zealand White rabbits after the oral administration of 600 IU anti-Xa/kg of bemiparin granules in gelatine capsules. SC corresponds to the administration of 150 IU/kg of Bemiparin dissolved in 1 ml of distilled water by subcutaneous route (n=3).

**Figure 13:** Release kinetics of enoxaparin from pellets with a diameter between 0.5 and 0.8 mm (above) and with a diameter comprised between 0.8 and 1.2 mm (below) (n=3).

**Figure 14:** Anti-Xa activity per ml in plasma of New Zealand White rabbits after the oral administration of 600 IU anti-Xa/kg of enoxaparin pellets in gelatine capsules. One group corresponds to pellets with a size comprised between 0.5 and 0.8 mm and another group corresponds to pellets with a size between 0.8 and 1.2 mm (n=3 $\pm$ standard deviation).

**Figure 15:** Distribution of the particle size of the granules obtained. The bars represent the % by mass of the different fractions obtained after the sieving process.

**Figure 16:** Release of Bemiparin from the granules obtained. The figure represents % of Bemiparin released over the total as a function of time. The dissolution medium is phosphate buffer at pH 6.8 (n=2; series 1 and 2).

**Figure 17:** Anti-Xa activity per ml in plasma of New Zealand White rabbits after the oral administration of 600 IU anti-Xa/kg bemiparin granules in gelatine capsules. The groups correspond to granules with percentages of coating of the polymer mixture of 8, 4 and 2.5% respectively (n=3). 0% corresponds to the administration of primary granules (uncoated) in hard gelatine capsules at a dose of 600 IU/kg. SC corresponds to the administration of 150 IU/kg of Bemiparin dissolved in 1 ml of distilled water by subcutaneous route (n=3).

**Figure 18:** Anti-Xa activity per ml in plasma of New Zealand White rabbits after the oral administration of 600 IU anti-Xa/kg bemiparin granules in gelatine capsules. The groups correspond to granules with percentages of coating of the polymer mixture of 4% which are incorporated respectively in non-enteric and enteric gelatine capsules (n=3). SC corresponds to the administration of 150 IU/kg of Bemiparin dissolved in 1 ml of distilled water by subcutaneous route (n=3).

**Figure 19:** Distribution of the particle size of the granules produced. The bars represent the % by mass of the different fractions obtained after the sieving process.

**Figure 20:** Anti-Xa activity per ml in plasma of New Zealand White rabbits after the oral administration of 600 IU anti-Xa/kg of bemiparin granules in gelatine capsules. The groups correspond to granules coated with a mixture of Eudragit RS and PCL in 9:1 proportion (n=3). SC corresponds to the administration of 150 IU/kg of Bemiparin dissolved in 1 ml of distilled water by subcutaneous route (n=3).

**Figure 21:** Distribution of the particle size of the granules produced. The bars represent the % by mass of the different fractions obtained after the sieving process.

**Figure 22:** Anti-Xa activity per ml in plasma of New Zealand White rabbits after the oral administration of 600 IU anti-Xa/kg of bemiparin granules in gelatine capsules. The groups correspond to granules with or without subcoating with Eudragit L and coated by a mixture of Eudragit RS and PCL in 1:1 proportion (n=3). SC corresponds to the administration of 150 IU/kg of Bemiparin dissolved in 1 ml of distilled water by subcutaneous route (n=3).

**Figure 23:** Distribution of the particle size of the granules obtained. The bars represent the % by mass of the different fractions obtained after the sieving process.

**Figure 24:** Release of Bemiparin from the granules obtained. The figure represents % of Bemiparin released over the total as a function of time. The dissolution medium is phosphate buffer pH 6.8 (n=2; series 1 and 2).

**Figure 25:** Anti-Xa activity per ml in plasma of New Zealand White rabbits after the oral administration of 600 IU anti-Xa/kg of bemiparin granules in gelatine capsules. The granules have on the surface a Eudragit RS and Pluronic F68 matrix in 1:1 proportion (n=3). SC corresponds to the administration of 150 IU/kg of Bemiparin dissolved in 1 ml of distilled water by subcutaneous route (n=3).

**Figure 26:** Anti Xa activity by ml in New Zealand White rabbit plasma upon oral adminstration of Bemiparin suspension according to the formulations obtained in example 20 (600 IU of anti Xa/kg) (squares: suspension of Bemiparin in a dissolution of Eudragit RS PO and PCL in acetone, circles: suspension of Bemiparin in acetone). (n=2). The triangles serie corresponds to the administration of 150 IU/kg of Bemiparin dissolved in 1 ml of distilled water by subcutaneous route (n=3).

## DETAILED DESCRIPTION OF THE INVENTION

As used in the description, the following terms have the meaning indicated below, unless otherwise stated:

"Polymeric matrix" means the structure comprising a solid or semisolid mass of polymer or a polymer mixture, either alone or mixed with one or more excipients.

"In contact" means that, in the pharmaceutical form, the active compound is either intimately mixed with the polymer matrix (full contact) or it is partially mixed with the polymeric matrix, for instance when the core (in which the active compound is present) is overcoated by a composition comprising the polymeric matrix (partially in contact)

"Non extended release form or formulation" is a pharmaceutical form or formulation that releases the active compound immediately after dosing and does not allow a reduction in dosage frequency. The pharmaceutical form or formulation not contains sustained release polymers.

"Poorly permeable hydrophilic molecule" refers to a molecule with affinity for aqueous media and which presents a low absorption through mucous membrane in a determined site. Thus, for example, hydrophilic molecules with a negative charge at pH greater than or equal to 4 will have difficulty to pass through the digestive tract mucosa which is coated by negatively charged mucus and which has a natural tendency to repel molecules with the same charge.

"Biodegradable" refers to a material that can be degraded into the body, so that is generally not eliminated intact.

"Granules" are understood as formulations constituted by agglomerates of particles or powders of small size, which may have an irregular or spherical shape. Granules are used as intermediate products in the manufacture of a pharmaceutical form, although they may also be used as end product.

"Core" is the inner part of a granule or a pellet and contains the active principle. The active compound in the core can either be dispersed on the whole mass of the core or can be forming a coating layer on an inert seed.

"Inert seed" is a bulk substance (e.g. microcrystalline cellulose, starch, sucrose or any other inert material) with spherical or not spherical form intended to be used as substrate for further coatings.

"Pellet" is understood as that material which is compacted in the form of small spheres or cylinders by procedures such as compaction, extrusion and/or spheronization (fusion of the solid mass, wetting of the dry mass, extrusion of the wet or melted mass and rotation of the extrudate by spheronization and subsequent drying). The pellet may contain the active compound therein or serve as support for the addition of the active compound on its surface.

In the definition of the process for the preparation of a pharmaceutical form, the term "combining" means that the active compound can or not be put in contact with the polymer of cationic nature, e.g., the active compound may be mixed with the polymer of cationic nature or may be forming a "core", optionally separated from the cationic polymer by an intermediate layer.

"Conditions that a polymeric matrix is formed" means conditions wherein a solid or semisolid mass of a polymer or polymer mixture and optionally one or more excipients, containing or not at least one active compound is formed (e.g. granulation, extrusion, layering, etc.)

"Maximum sink" gradient conditions are conditions for the determination of active substance release in vitro at which the maximum concentration of active compound which can be released in a release medium is lower to 30% of the saturation concentration, with the aim that the concentration gradient can be considered not limiting.

"Biomaterial" includes all materials capable of being in contact with body tissues with specific therapeutical, diagnostic or preventive purpose. These materials must be biocompatible.

"Biocompatible" refers to a material which causes no significant adverse response of the physiological medium after interacting with body tissues and fluids and, in cases, it must biodegrade, chemically as well as physically, or by a combination of the two, into no-toxic components.

One first aspect of the invention relates to a pharmaceutical form containing at least an active compound and a polymeric matrix, wherein said polymeric matrix comprises at least one polymer of cationic nature. According to a preferred embodiment, the polymeric matrix form part of the surface of the pharmaceutical form.

In an embodiment, in the pharmaceutical form of the invention the polymeric matrix is in contact with the active principle.

In a preferred embodiment of the pharmaceutical form of the invention, the matrix comprises at least one polymer of cationic nature and at least another polymer of anionic or neutral nature. Additionally, the matrix may contain compounds selected from plasticizers, glidants, absorption enhancers, humectants, surfactants, colouring agents and dispersants.

Preferentially, in the pharmaceutical form of the invention the release of the active compound is of at least 30 % in a time lower than or equal to 60 minutes, more preferably at least 50% in a time lower than or equal to 60 minutes

The particle size of the pharmaceutical form of the invention preferentially is at least 0,1mm

In an embodiment of the invention, the pharmaceutical form is a non extended release form.

Preferentially, the active compound in the pharmaceutical form of the invention is a poorly permeable hydrophilic molecule containing groups with negative charges at pH greater than or equal to 4. Particularly, it is selected from the group consisting of antibiotics, anti-inflammatories, anti-infectious agents, antiparasitics, hormones, substances with immunological activity, vaccines, immunomodulators, immunosuppressants, cytostatics, diuretics, agents with activity in the digestive system, agents with activity in the circulatory system, agents with activity in the respiratory system, human growth hormone, recombinant growth hormone, bovine growth hormone, growth-hormone releasing hormone, interferons, analgesics, agents with activity in the central nervous system, erythropoietin, somatostatin, gonadotropin-releasing hormone, follicle-stimulating hormone, oxytocin, vasopressin, parathyroid hormone, adrenocorticotropin, gonadotropin-releasing hormone, thrombopoietin, calcitonin, interferons, interleukins, insulins, unfractionated heparin, low molecular weight heparin, ultra low molecular weight heparins, heparinoids, dermatan, glucosamines, chondroitins, auricular natriuretic factor, monoclonal antibodies, protease inhibitors, filgrastim, prostaglandins (PGE2 and PGI2), cyclosporin, cromolyn sodium, cromoglycate and its salts, vasopressin, vancomycin, neomycin, desferrioxamine, antimicrobial agents, antifungals, cytostatics, immunomodulators, vitamins, antivirals, antigens, ribonucleic acid, deoxyribonucleic acid, oligonucleotides, CPG sequences, plasmids, active compounds of protein nature, active compounds of polysaccharide nature, glucocerebrosidase, and derivatives and combinations thereof. More particularly, it is selected fron the group consisting of insulins, unfractionated heparin, low molecular weight heparin, ultra low molecular weight heparins and heparinoids, even more particularly it is low molecular weight heparin.

For the purposes of the present invention: "Low Molecular Weight Heparin" is a sulphated glycosaminoglycane salts with an average molecular weight less than 8000 Da and with a 60 % of the molecules having a molecular weight less than 8000. These molecules have different chemical structures as well as different reducing and non-reducing ends of the polysaccharide chains. Anti-factor Xa activity is not less than 70 IU per milligram of dry substance. Anti-factor Xa to anti-factor IIa is not less than 1.5. "Ultra-Low Molecular Weight Heparin" is a sulphated glycosaminoglycane salts with an average molecular weight less than 4000 Da. These molecules have different chemical structures as well as different reducing and non-reducing ends of the polysaccharide chains. Anti-factor Xa activity is not less than 70 IU per milligram of dry substance. Anti-factor Xa to anti-factor IIa is not less than 10.

According to an embodiment of the pharmaceutical form of the invention, at least one polymer of anionic or neutral nature and at least one polymer of cationic nature are present in a proportion of at least 10 % each in relation to the weight of the polymeric matrix.

In a particular embodiment of the invention, the polymer of cationic nature in the polymeric matrix of the pharmaceutical form is selected from polymers and copolymers derived from acrylic and methacrylic acids, and natural polymers.

Particularly, the polymer of anionic or neutral nature in the polymeric matrix of the pharmaceutical form is selected from the group consisting of: polyesters, polycaprolactones, polymers and copolymers derived from acrylic acid, polyethylene oxides, polypropylene oxides, polyethylene and polypropylene oxide copolymers, polyanhydrides, polyamides, polyurethanes, polycarbonates, polyacetals, polyorthoesters, polycyanacrilates, polydioxanones, poly ($\alpha$-hydroxy acids), poly ($\beta$-hydroxy acids) polyphosphazenes, natural polymers; mixtures, copolymers and terpolymers thereof. More particularly, the polymers of anionic or neutral nature the polyesters are selected from the group consisting of: lactic acid polymers, glycolic acid polymers, lactic and glycolic acid copolymers; the polycaprolactone is poly-$\varepsilon$-caprolactone; the acrylic acid derived polymers are selected from the group of polymethylmethacrylates; and the natural polymers are selected from the group of cellulose derivatives formed by microcrystalline cellulose, hydroxypropylmethylcellulose and ethyl cellulose; mixtures, copolymers and terpolymers thereof. Even more particularly the polymers of anionic and neutral nature in the polymeric matrix are biodegradable.

In an embodiment of the pharmaceutical form of the invention the polymeric matrix comprises polyesters and natural biodegradable polymers as polymers of anionic or neutral nature and methacrylic acid derivatives with quaternary ammonium groups as polymers of cationic nature. Particularly, the polymeric matrix comprises poly-$\varepsilon$-caprolactone as a polymer of anionic or neutral nature and a polymer derived from methacrylic acid with quaternary ammonium groups as a polymer of cationic nature. More particularly, the polymer derived from methacrylic acid with quaternary ammonium groups is a trimethylammonioethyl methacrylate chloride copolymer.

In other embodiment, the polymeric matrix of the pharmaceutical form comprises a polyethylene and polypropylene oxide copolymer as polymer of neutral nature and a polymer derived from methacrylic acid with quaternary ammonium groups as polymer of cationic nature.

Among the solid forms used for the administration of active compounds, granules, coated granules and pellets are included. Therefore, according to an embodiment, the pharmaceutical form of the invention is a granule and, in another embodiment, it is a pellet.

When the pharmaceutical form of the invention is a granule, in an embodiment, the granule comprises a core and a polymeric matrix, wherein said core comprises at least an active compound and said polymeric matrix comprises at least one polymer of cationic nature. The polymeric matrix further may comprise at least one polymer of anionic or neutral nature.

In a particular embodiment, the polymeric matrix forms part of the core of the granule.

In another particular embodiment, the polymeric matrix in the granule forms a coating layer on or over the core. In special cases, for instance, when the polymeric matrix exerts an undesirable effect on the active compounds, the presence of an intermediate coating layer between the active compounds and the polymeric matrix is useful in order to avoid the contact. Intermediate coating layer may be constituted by any substance providing physical separation between the core and the polymeric matrix on surface. Examples of coating substances can be but not limited to: cellulose derivatives, copolymers of acrylic or methacrylic acid polyethylene oxides, polypropylene oxides, polyethylene and polypropylene oxide copolymers, gelatin, lactose, mannitol. Intermediate coating layer may also contain any excipient commonly used in surface coating such as glidants and plasticizers. According to a variant, the core in the granule may comprise an inert seed wherein the active compound form part of a layer coating said seed. In this case, the core also may comprise an intermediate layer between the inert seed and the layer comprising at least an active compound. This intermediate layer is useful for sealing the inert seed in order to avoid the deleterious effect of moisture. Therefore, this layer comprises any compound intended to protect from moisture e.g. ethyl cellulose, hydroxypropyl methyl cellulose, neutral polymers derived from methacrylic acid e.g. Eudragit NM or Eudragit NE, poloxamers and high molecular weight polyethyleneglicols (e.g. PEG 8000), polyvinyl acetate phthalate, cellulose acetate phthalate and waxes.

When the pharmaceutical form of the invention is a pellet, it comprises at least one active compound and a polymeric matrix, wherein said polymeric matrix comprises at least one polymer of cationic nature. The polymeric matrix further may comprise at least one polymer of anionic or neutral nature.

When the pharmaceutical form is a liquid it comprises at least one active principle in combination with a dissolution of a polymer in a pharmaceutical solvent, wherein the polymer dissolution comprises at least one polymer of cationic nature. The liquid form can be e.g. a suspension of at least one active principle in a dissolution of a polymer in a pharmaceutical solvent, wherein the polymer comprises at least one polymer of cationic nature.

With regard to the pharmaceutical forms protected by the present invention, there are different processes whereby they can be manufactured or produced.

By way of a non-limitative and illustrative example, two general procedures are cited for the obtention of granules or pellets which are sufficiently known by any person skilled in the art:

Dry granulation:

It is produced by the aggregation of the powder components subjected to high pressure followed by a fragmentation or breaking into pieces and subsequent separation by sieving to achieve the desired granule size. The techniques more commonly used are slugging and roll compaction *[Roll compaction/dry granulation: pharmaceutical applications. Kleinebudde, Peter. European Journal of Pharmaceutics and Biopharmaceutics (2004), 58(2), 317-326]*.

Wet granulation:

It consists of a kneading process of a powder mixture in the presence of granulation liquid, water or organic solvent, which can have a binding agent dissolved or be incorporated with the other mixture components to favour the adhesion of particles once the granules have dried *[Theophylline granule formulation prepared by the wet granulation method: comparison of in vitro dissolution profiles and estimation of in vivo plasma concentrations. Karasulu et al. European Journal of Drug Metabolism and Pharmacokinetics (2007), Volume 31(4), 291-298]*.

A variant of this procedure is granulation in fluidized bed, wherein the granulation liquid is sprayed on the particles suspended in air current *[Experimental study of wet granulation in fluidized bed: Impact of the binder properties on the granule morphology. Rajniak, P et al. International Journal of Pharmaceutics (2007), 334(1-2), 92-102]*.

This procedure also permits coating preformed granules as well as obtaining granules from inert seeds on which a suspension or dissolution of the active compound is applied [Preparation and evaluation of sustained release indomethacin nonpareil seeds. The-Mahrouk et al. Drug Development and Industrial Pharmacy (1993), 19(15), 1903-16]

**EP 2 060 253 A1**

for example, nonpareil starch or sugar cores or microcrystalline cellulose spheres. An additional variant of this technique is the production of pellets by the spheronization procedure [The preparation of pellets containing a surfactant or a mixture of mono- and di-glycerides by extrusion/spheronization. Newton, et al. European Journal of Pharmaceutical Sciences (2007), 30(3-4), 333-342].

Therefore, according to a second aspect, the invention relates to a process for the preparation of the pharmaceutical forms aforementioned. Said process comprises combining at least an active compound and at least one polymer of cationic nature in conditions that a polymeric matrix is formed.

According to an embodiment, the process described in the previous paragraph comprises the step of subjecting a composition comprising at least one active compound to a granulation process to produce a core. Particularly, the composition further comprises at least one polymer of cationic nature. More particularly, the composition further comprises at least one polymer of anionic o neutral nature. Even more particularly, the process further comprises the step of coating the core with a composition comprising at least one polymer of cationic nature, and preferentially, the composition of the coating further comprises at least one polymer of anionic or neutral nature.

According to another embodiment, the process for preparing a pharmaceutical form of the invention comprises the steps of :

a) coating an inert seed with a composition comprising at least one active compound to obtain a core, and

b) coating the core prepared in a) with a composition comprising at least a polymer of cationic nature.

In a variant, the process further comprises in step a) coating the inert seed with a composition to form an intermediate coating layer before performing the coating with a composition comprising at least one active principle.

Particularly, the composition used in step b) further comprises at least one polymer of anionic or neutral nature. More particularly, a spray method is used in the coating performed in step b)

The processes described before may further comprise the step of coating the core with a composition to form an intermediate coating layer. As was mentioned above in this description, the presence of an intermediate layer protects the active compound when it can be affected by the polymers comprised in the matrix.

According to a further embodiment, the process for preparing a pharmaceutical form of the invention comprises the steps of

a) mixing at least one active compound with at least one polymer of cationic nature, and

b) extruding the homogenized mixture.

Preferentially, the active compound is further mixed with at least one polymer of anionic or neutral nature in step a).

When the pharmaceutical form is a liquid, one method for its manufacture can be, for instance, as follows:

a) preparing a dissolution comprising at least one polymer of cationic nature in a pharmaceutically acceptable dissolvent

b) preparing a dissolution of at least one active principle in a pharmaceutically acceptable solvent

c) combining the dissolutions obtained in a) and b)

In a particular case the liquid form may be a suspension.

Particular preparation processes of the pharmaceutical forms of the invention are the following:

1. Preparing granules comprising a core of active compound and microcrystalline cellulose with a coating representing a weight gain with respect to the uncoated granule of 4% and wherein the coating composition is selected from:

a.- Eudragit RS and polycaprolactone in a 1:1 ratio dissolved in acetone

b.- Eudragit RS and PLGA in 1:1 ratio dissolved in an organic solvent

c.- Eudragit RS and polyethylene and propylene oxide copolymer in a 1:1 ratio dissolved in acetone

d.- Eudragit RS and Aquacoat in 1:1 ratio in aqueous suspension

2. Preparing granules of group 1 which additionally includes applying an intermediate coating of cellulose derivatives (cellulose acetate phthalate or hydroxy propyl methyl cellulose phthalate), or Eudragit L or S to confer grastroresistance and non-gastric release.

3. Preparing granules obtained by wet granulation of carboxymethylcellulose and heparin dissolved in water subjected to secondary granulation with Eudragit RS.

4. Preparing granules by wet granulation of microcrystalline cellulose dissolved in water and aqueous suspension of Eudragit RS wherein the microcrystalline cellulose:Eudragit RS is 1:0.8.

5. Preparing a heparin pellet by extrusion/spheronization of microcrystalline cellulose and Eudragit RS and 2.66:1 ratio.

The pharmaceutical form according to the present invention may be subsequently processed to obtain a pharmaceutical formulation which is the final product to be administrated to a patient. The features of the pharmaceutical formulation depend on the route of administration, particular release profiles, or other intended effects.

For example, the granules and pellets thus produced can be designed for the preparation of tablets and/or capsules *[Characterization of 5-Fluorouracil Release from Hydroxypropylmethylcellulose Compression-Coated Tablets. Wu et al. Pharmaceutical Development and Technology (2007), 12(2), 203-210]*, or they can be subject to additional treatments such as polymeric coatings of granules produced to give:

- Gastro-resistance: *[In vitro dissolution studies of sodium diclofenac coated granules with eudragit L-30D-55 by fluidized-bed system. Silva, OR. et al. Drug Development and Industrial Pharmacy (2006), 32(6), 661-667]*, such as, for example, polyvinyl acetate phthalate, hydroxypropyl methylcellulose phthalate, acrylic copolymers as methacrylic acid and methacrylic ester copolymers, hydroxypropylmethylcellulose acetate succinate, etc.,

- Mucoadhesiveness: such as, for example, chitosan or,

Therefore, a third aspect of the invention provides a pharmaceutical formulation comprising a pharmaceutical form as described above. Preferentially, said pharmaceutical formulation is a non extended release formulation. Particularly, the formulation takes the form of a tablet, a capsule, a granule, a pellet, a lyophilisate, a serum, a microparticle, an oily suspension, a liposome, a micelle, a self-emulsifying system and other dispersed systems.

Oral administration continues being an attractive route for the release of pharmacologically active molecules. Its easy administration, the absence of pain associated to the administration, the greater acceptance by the patient, and favourable cost/benefit ratio have turned these oral formulations into the most widely used for the administration of active compounds by oral route. Therefore, in a preferred embodiment the pharmaceutical formulation of the invention is for administration by oral route.

A forth aspect of the invention is the use of the pharmaceutical form, as described above for the preparation of a pharmaceutical formulation. Particularly, the pharmaceutical formulation is not an extended release formulation.

A fifth aspect of the invention refers to the use of a pharmaceutical form or a pharmaceutical formulation as defined above, in the preparation of a medicament applicable to mucosas selected from oropharyngeal mucosa, gastrointestinal mucosa, pulmonary mucosa, nasal mucosa and vaginal mucosa.

A sixth aspect of the invention relates to the use of the afore mentioned pharmaceutical form or pharmaceutical formulation as a medicament applicable to mucosas selected from the group formed by oropharyngeal mucosa, gastrointestinal mucosa, pulmonary mucosa, nasal mucosa and vaginal mucosa.

By way of summary, the results disclosed by the present invention favours the absorption of molecules, particularly, those whose systemic bioavailability is more limited by the reduced permeability in the absorption barrier of the molecule, due to the reduced solubility thereof in the luminal fluids by mucosal route, preferably administered by oral route, especially for molecules which in the state of the art are commonly known for their low and/or erratic absorption by oral route.

The pharmaceutical form object of the present invention achieves yields of incorporation of the active compound of at least 80%, which is an advantage compared with the microparticle and/or nanoparticle formulations.

*EXAMPLES*

The following examples illustrate the invention and should not be considered in a limitative sense thereof.

**Heparins as active compounds of interest.**

Heparins are anticoagulants which act by the inactivation of certain coagulation cascade factors. Heparins are essentially used for their anticoagulant (related with the inhibition of factor IIa) and antithrombotic (by the inhibition of factor Xa) properties for the prevention and the treatment of thromboembolic diseases *[Low- and ultra-low-molecular-weight heparins. Best Pract Res Clin Haemal. 2004; 17: 77-87]*:

- in prevention, to reduce the incidence of thromboembolic complications after prolonged immobilization due to a disease, and after surgical interventions *[Prevention of venous thromboembolism. Agnelli and Sonaglia., Tromb Res. 2000, 97: V49-62]*.

- in curing, for the treatment of deep vein thrombosis *[Treatment of venous thromboembolism. Ageno. Tromb Res. 2000, 97: V63-72.]*, of pulmonary embolisms, of disseminated intravascular coagulation, acute arterial obstruction and the acute phase of myocardial infarction.

Currently, heparin is extracted from porcine or bovine intestinal mucosa *[Heparins: all a nephrologist should know. Hetzel et al. Nephrol Dial Transplant. 2005; 20: 2036-42]* the unfractionated heparin is a heterogeneous mixture of sulfated mucopolysaccharide chains whose molecular mass is between 3,000 and 30,000 daltons. Its average molecular mass is 15,000 Daltons, and it corresponds to a heparin molecule of around 45 osidic units *[Molecular weight dependency of the heparin potentiated inhibition of thrombin and activated factor X. Effect of heparin neutralization in plasma. Andersson et al. Thromb Res. 1979; 15: 531-41]*.

If a chemical or enzymatic depolymerisation is performed, heparins consisting of shorter chains, and consequently, with a lower molecular mass, between 1,000 and 10,000 daltons are produced. Their average molecular mass is 4,500 daltons. These heparins, called low molecular weight heparins (LMWH), are then distinguished from unfractioned heparins (UH) by a predominantly anti-Xa activity.

Heparin acts by the intermediation of a cofactor: antithrombin III (ATIII), which is a natural plasma inhibitor of coagulation *[Heparin and Low-Molecular-Weight Heparin: The Seventh ACCP Conference on Antithrombotic and Thrombolytic Therapy. Hirsh and Raschke. Chest. 2004; 126: 188S-203S]* and it behaves as a catalyst with respect to ATIII. ATIII has a slow and progressive action. Once the heparin has been fixed to the ATIII by the intermediation of the pentasaccharide fragment, this action becomes immediate. This fixation causes a change in the formation of the ATIII which then permits the irreversible fixation thereof on the active site of serine proteinase-type coagulation factors (factors IIa, Xa and IXa, mainly). Then, heparin is released intact, and can then react with a new antithrombin molecule.

It should be mentioned that the pharmacodynamic effect of heparins depends on the chain length of oligosaccharides. Indeed, to inhibit the thrombin, heparin should be fixed on ATIII and on thrombin through a pentasaccharide block. On the other hand, to inhibit factor Xa, heparin should only fix to ATIII by the pentasaccharide block. Thus, the fragments with a molecular mass (MM) below 5,400 Da, i.e. 18 saccharide units, lose their capacity to be simultaneously fixed to thrombin and ATIII, and will thus have an essentially anti-Xa activity. The fragments with a MM greater than or equal to 5,400 Da will be both anti-Xa and anti-IIa. Standard heparin comprises fragments with a variable molecular mass of 2,000 to 30,000 Da, and therefore has activity on the two factors, Xa and IIa *[Heparin and Low-Molecular-Weight Heparin: The Seventh ACCP Conference on Antithrombotic and Thrombolytic Therapy. Hirsh and Raschke. Chest. 2004; 126: 188S-203S]*. It therefore has both an antithrombotic and an anticoagulant activity, in comparison with low molecular weight heparins, which are essentially antithrombotic agents with a predominance of anti-Xa activity.

Venous thromboembolic disease continues to be responsible for important morbidity and and/or mortality. In fact, in the United States, the number of persons hospitalized for this reason is estimated at between 300,000 and 600,000 per year. Furthermore, this disease, due to the pulmonary embolism of which it is the cause, would be responsible for 50,000 *[Development of oral heparin therapy for prophylaxis and treatment of deep venous thrombosis. Money and Gorka. Caridovasc Surg.2001; 9: 211-8]* to 100,000 deaths per year in the United States *[Prevention of venous thromboembolism. Agnelli and Sonaglia. Tromb Res. 2000, 97: V49-62]*.

Additionally, pulmonary embolism can be produced without clinical venous thrombosis, henceforth the interest in prevention with regard to patients with a risk of thrombosis, among which are: cancer, age over 70, prolonged immobility, paralysis, obesity or even taking estrogens by oral route.

Since heparin acts on the coagulation factors by a catalysis mechanism mediated by ATIII, the measurement of its plasma concentration does not constitute an efficient means to determine its biological activity.

The procedures used should instead reflect heparin's capacity to inhibit factors Xa and IIa. For this reason, different measurement process can be used in human beings and animals:

- the measurement of the coagulation factor activity, expressed in units of inhibition of Xa or IIa activity.

-  the measurement of the haemorrhaging time determined by the activated partial thromboplastin time (aPTT). This test explores the intrinsic route of the coagulation measuring the coagulation time of decalcified and platelet-impoverished plasma in the presence of a platelet equivalent (cephalin) and calcium.

-  the measurement of the prothrombin time exploring the intrinsic coagulation route; this procedure uses recalcified blood plasma in the presence of tissue thromboplastin [Digestive absorption of heparin with alternative formulations. Ubrich et al., 2002. S.T.P. Pharma Sciences. 2002; 12: 147-55].

There is another process which only exists in animals, and consists of measuring the variation in the size of a thrombus. This does not permit quantifying the response, but it shows if there is a proven pharmacological action of the anticoagulant.

**Insulin as active compound of interest**

Insulin is the "anabolic" hormone par excellence; i.e. it permits providing the cells with the sufficient supply of glucose for the synthesis process with energy expenditure, which will then, through glycolysis and cell respiration, produce the necessary energy in the form of ATP (adenosine triphosphate mononucleotide) which uses the metabolism as unit of transportable energy for said processes.

It maintains the glucose concentration in our blood. It achieves this because when the glucose level is high the pancreas releases it into the blood. Its function is to favour the cell absorption of glucose.

It is one of the two hormones produced by the pancreas together with glucagon (unlike insulin, when the glucose level decreases it is released into the blood). Insulin is produced in the pancreas in the "Islets of Langerhans", by cells called Beta cells. One way of detecting if the Beta cells produce insulin is by carrying out a test, checking for the presence of C-peptide in the blood. C-peptide is released into the blood when the Beta cells process the proinsulin, converting it into insulin. Only when between 10% and 20% of the Beta cells are in good condition, symptoms of diabetes begin to appear, first passing through a prior state called honeymoon where the pancreas still secretes some insulin.

Normally, the insulins currently used are synthesized by means of genetic engineering through recombinant DNA technology, due to the fact that a rapid, medium or slow activity is pursued.

Types of insulins:

In type I diabetes, and in some cases of type II, it is necessary to inject insulin to maintain a correct glucose level in the blood. The following types of insulins exist:

-  Quick-acting insulins.
-  Intermediate-acting insulins or NPH.
-  Slow-acting insulins.
-  24-hour insulins.

By injection areas. We can also classify them by their injection areas as:

-  Subcutaneous insulins: any insulin, whether quick-acting or delayed
-  Endovenous insulins: only quick-acting insulins which do not have retarders.
-  Inhaled insulin: there is currently a new type of insulin, inhaled insulin, which despite being as efficient as the traditional one, eliminates the need of being injected to the patient.

However, there is no insulin on the market which can be administered by oral route, which makes our solid formulation extremely interesting.

**Example 1: Formation of granules with heparin.**

The heparin granules have been prepared by dispersing a heparin solution in microcrystalline cellulose (MCC) in order to produce primary granules which, then, have been mixed with the aid of an organic solution of Eudragit® RS (ERS) and poly-ε-caprolactone (PCL) both in equivalent quantities.

As an alternative procedure, the heparin granules have been prepared by wetting the polymers with an aqueous solution containing heparin. The first stage consists of dispersing a heparin solution within a microcrystalline cellulose powder. The primary granules, produced during this stage, are then remixed with the aid of an aqueous suspension of Eudragit® RS and ethyl cellulose.

*In vitro* studies have been performed in order to determine the manufacturing yields and the water contents of the manufactured granules, and of evaluating the *in vitro* release kinetics of the active compound. Finally, a preliminary study has been performed *in vivo* on New Zealand White rabbits after oral administration of these heparin granules, in order to evaluate the absorption and oral bioavailability of heparin.

## Example 2: Formation of granules with Enoxaparin

Enoxaparin is a low molecular weight heparin (4,500 ± 1,000 Daltons) whose antithrombotic and anticoagulant activities are dissociated. It is characterized by higher anti-Xa activity than anti-IIa activity, the proportion between both activities being 3.6.

The form used for our experiments is an aqueous solution of LOVENOX® dosed at 10,000 IU of anti-Xa / mL and commercially designed for the subcutaneous route.

The polymers used for the creating the polymeric matrix are:

Cellulose:

Cellulose is a natural homopolysaccharide formed by units of α-D-glucopyranose bound together by a 1-4 glucosidic bond. There are intra and intermolecular hydrogen bridges within the molecule, and the immediate consequence of this structural characteristic is the insolubility of the cellulose both in classic organic solvents and in water. Cellulose is widely used in the pharmaceutical industry as excipient in direct compression.

Eudragit® RS 30 D:

Eudragit® RS 30 D, kindly provided by Degussa (Darmstadt, Germany), is a copolymer of trimethylammonioethyl methacrylate chloride which has an overall positive charge with a low concentration in quaternary ammonium groups (from 8 to 12%). This polymer is a nonbiodegradable synthetic polymer. It is not absorbed nor metabolized, for which reason it is eliminated intact. It is insoluble in water but soluble in organic solvents. It is presented in the form of an aqueous suspension at a polymer concentration of 30%(m/v).

Aguacoat® ECD:

Aquacoat® ECD, kindly provided by FMC BioPolymer (Newark, DE, USA.), corresponds to a suspension of a synthetic polymer of ethyl cellulose, non-ionic, insoluble in water but soluble in organic solvents. Aquacoat® ECD is used in the pharmaceutical industry for the manufacturing of granules and matricial systems, as well as for coating tablets. It is also available in the form of an aqueous suspension at a polymer concentration of 30%(m/v).

The procedures used to granulate are described below:

**Primary Granulation Stage**

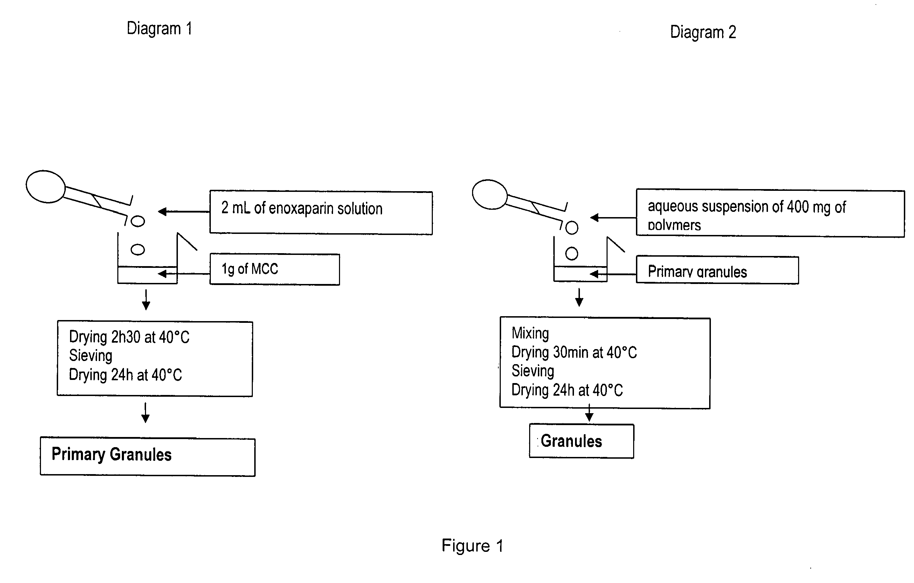

The first stage, called primary granulation, consists of mixing the aqueous solution of enoxaparin (2 mL at 2,000, 3,000, 4,000 or 5.000 IU of anti-Xa) with 1 g of microcrystalline cellulose (MCC). After drying for 2 hours 30 in the oven at 40˚C, the agglomerates are forced to pass through a metal sieve with a mesh opening of 600 μm. The primary granules thus produced are then dried for 24 hours in the oven at 40˚C (Figure 1, Diagram 1).

**Addition of polymers**

The second stage, of regranulation, consists of adding an aqueous suspension of polymers to the primary granules. The primary granules are then directly mixed with different proportions (100/0, 75/25, 50/50, 25/75, 0/100) of aqueous suspensions of aquacoat® ECD (AqC) and/or Eudragit® RS 30 D (RS) corresponding to 400 mg of polymers. The product is dried in the oven at 40˚C for 30 minutes. Once dry, the agglomerates are forced to pass through the metal sieve with a mesh opening equal to 600 μm. The granules produced are dried in the oven at 40˚C for 24 hours (Figure 1, Diagram 2).

The "virgin" granules, without heparin, have been prepared in the same manner, substituting the heparin solution for water. The primary granules prepared with 500 and 1,000 IU of anti-Xa are also mixed with 100% Eudragit® RS 30 D.

Finally, the primary granules produced with 500 mg of MCC and 5,000 IU of anti-Xa of enoxaparin (2 mL), and then mixed with 100% Eudragit® RS 30 D, have also been formulated according to the same procedure.

Characterization of the granules produced:

*Morphology and size*

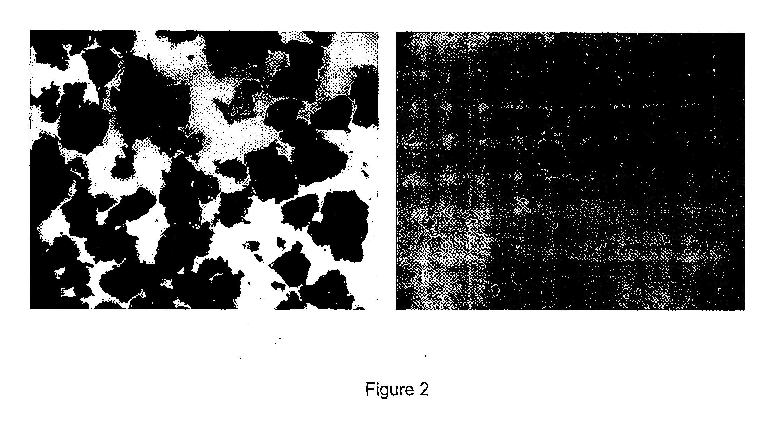

The granules have been observed under the optical microscope in these conditions [ Figure 2 (left)] and after having been dispersed in a 4% PVA solution [Figure 2 (right)]. After acquisition of the image, it is analysed by a computer programme (Kappa programme) which permits calculating the size.

*Manufacturing yield*

The preparation yield is calculated with respect to the initial masses of cellulose, the addition polymers and enoxaparin.

*Water content*

The water content of the different granules prepared has been obtained by potentiometry using the Karl Fisher method. The water content measurements have been made in duplicate for each sample. These measurements have been made with the aid of model 756 KF, Metrohm SA potentiometer (Herisau, Switzerland).
In order to optimize the drying time of the primary granules and of those containing polymer mixture, the water content of the granules without heparin has been measured over time after 2, 4, 8 and 24 hours of drying.
With regard to the water content of the heparin granules, it has been measured after 24 hours of drying.

*In vitro release kinetics*

The objective of this assay is to determine the quantity of enoxaparin released by the granules over time. This *in vitro* release should be performed in defined conditions called "sink", i.e. those wherein the maximum concentration of active compound which can be released in a release medium is lower than 30% of the saturation concentration, so that the concentration gradient can be considered non-limiting.
The release assays have been carried out in a water bath thermostatted at 37˚C, suspending with gentle magnetic stirring (200 rpm) 50 mg of each batch of enoxaparin granules in 20 mL of phosphate buffer (PBS, 0.011 M and NaCl, 0.15 M) at pH 7,4.
1.5 mL samples have been taken with the automatic pipette after 5, 10, 15, 30, 45 minutes, and after 1, 2, 4, 6, 8 and 24 hours. The volumes collected are substituted by 1.5 mL of fresh buffer in order to maintain a constant volume in the flasks. These aliquots are then filtered with a MILLIPORE filter with 0.22 μm porosity.
The quantities of enoxaparin released are evaluated by nephelometry, and then the curves which represent the release kinetics of enoxaparin in accordance with time are established. The release kinetics have been performed in duplicate for each one of the samples (n=2).

*Nephelometric determination*

The nephelometric determination method is based on the capacity of numerous sulfate and carboxylate groups of enoxaparin to complex with quaternary ammonium groups of a 0.1 % (m/v) cetylpyridinium chloride solution in the presence of an acetate buffer. The importance of the complex produced is evaluated by absorption spectrophotometry at 500 nm.
A calibration scale is made from a 10 IU/mL enoxaparin stock solution prepared in a 0.1 %% phosphate buffer.
Acetate buffer (500 μL)r and cetylpyridinium chloride (2 mL) are successively added to 500 μL of each enoxaparin concentration or samples to dose. The combination is incubated at 37˚C in a water bath for one hour and then the absorbance is determined at 500 nm with the aid of a spectrometer (Uvikon 922 spectrophotometer Kontron instruments).
The release proportion is estimated form the following formula:

$$\text{Proportion released (\%)} = \frac{[(\text{units released in time } t \times 20) + (\text{units released in } t\text{-}1 \text{ per mL})] \times 100}{(\text{units encapsulated per gram of polymer}) \times 0.05 \text{ g}}$$

### Example 3: *in vivo* studies of granules with Enoxaparin

Size 0 capsules containing enoxaparin granules have been administered at a dose of 600 IU/kg to male rabbits of New Zealand White breed after a 12 hour fast, but with free access to water. Blood samples have been taken (1.5 mL) from the marginal vein of the ear at regular time intervals after oral administration (2, 4, 6, 8, 10 and 24 hours). The blood is collected in citrated tubes (1 volume of sodium citrate per 9 volumes of blood). After centrifuging the blood samples at 3,000g during 10 minutes, the blood plasma is removed and the anti-Xa plasma activity is dosed with the aid of a STAGO automaton. Only the granules prepared with 1 g of MCC and 5,000 IU of anti-Xa and then mixed with Eudragit® RS 30 D, with Aquacoat® ECD and with a mixture 50/50 of the two polymers, have been tested.

Assay principle of anti-Xa activity:

The determination was carried out together with antithrombin III (ATIII) purified in order for the results not to depend on a possible lack in this protein. The principle is based in the in vitro inhibition of factor Xa by ATIII-heparin complexes by an amidolytic procedure on the chromogen substrate, according to Teien [Evaluation of an amidolytic heparin assay method: increased sensitivity by adding purified antithrombin III. Teien and Lie. Thromb Res. 1977; 10: 399-410].

ATIII + heparin $\rightarrow$ [ATIII-heparin]

[ATIII-heparin] + Xa (in known excess) $\rightarrow$ [ATIII-heparin-Xa] + residual Xa

residual Xa + chromogen substrate (CBS 31.39) $\rightarrow$ Para-nitroaniline

When factor Xa is fixed to a chromogen substrate, this releases para-nitroaniline, its quantity being inversely proportional to the quantity of heparin present in the initial medium. The para-nitroaniline is determined by absorption spectrophotometry at 405 nm.
A calibration scale of 0 to 0.8 IU of anti-Xa/mL is made after dilution of the enoxaparin stock solution in the Orwen Koller buffer (Stago). The measurements of the points on the scale and the plasma samples are carried out with a Strachrom kit and with the aid of a STA automaton (Stago Diagnostica).

### Example 4: Calculation of the relative bioavailability of granules with Enoxaparin

Bioavailability is a parameter which characterizes the proportion and rate at which an active compound is absorbed in the organism with respect to the dose administered. Normally, it is characterized by the monitoring of the unaltered molecule in the blood. The comparison of the areas under the curves after oral and subcutaneous administrations leads to the relative bioavailability, which discloses the quantity absorbed by oral route with respect to the quantity absorbed by subcutaneous route, corrected by the dose administered. The rate parameter is generally measured indirectly, measuring the time until the maximum time (Tmax) and the maximum concentration (Cmax).
The relative bioavailability is calculated according to the formula:

$$\text{Relative bioavailability} = \frac{\text{area under the curve, oral x subcutaneous dose}}{\text{area under the curve, subcutaneous x oral dose}}$$

### Example-5: Calculation of the Manufacturing yield of granules with Enoxaparin

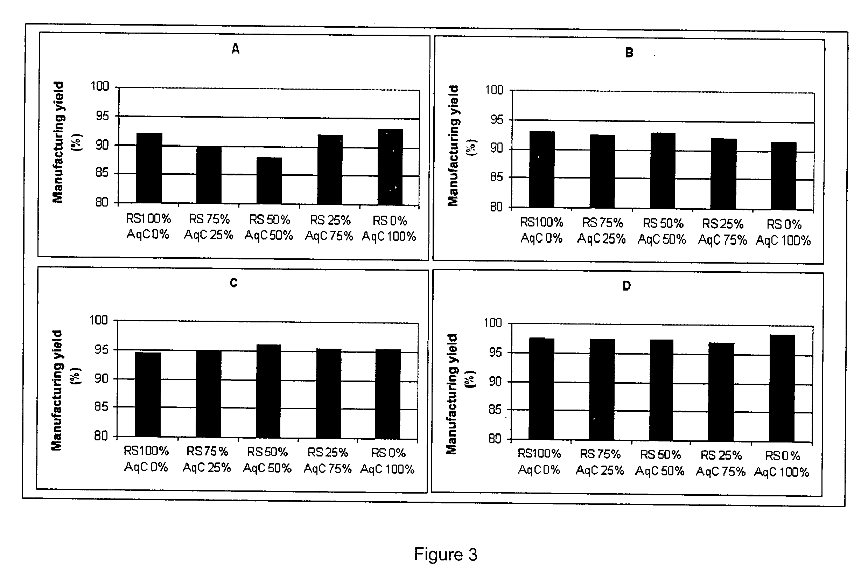

The average manufacturing yields of each granule formulation are indicated in figure 3 and vary between 90% and 99%. However, they are slightly lower and are between 88% and 92% for the granules prepared with 2,000 IU of heparin anti-Xa (figure 3) with more significant standard deviations.

Water content of the granules containing polymer mixture

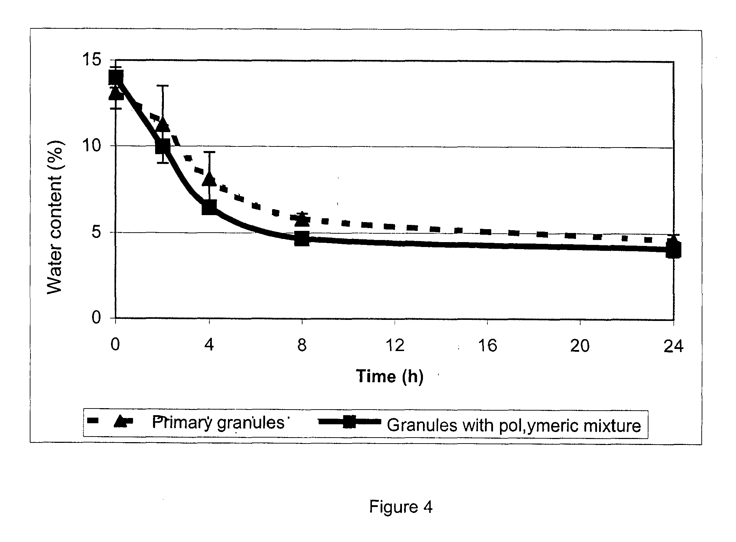

Figure 4 logically shows that the water content decreases over time during the drying stage of the primary granules and of those mixed with Eudragit® RS 30 D and Aquacoat® ECD 50/50, with little significant differences between the primary granules and the coated granules. An average water content of around 4% is obtained 24 h after drying for the two types of granules, with little difference between 8 h (from 4 to 6%) and 24 h (from 3 to 5%) of drying.

The average water content for each form of enoxaparin granules prepared with different quantities of LMWH and later mixed with different proportions of polymer is indicated in figure 4. It is located between 2 and 4%, whatever the quantity of heparin and the polymer or polymers added.

This preliminary study has been performed in order to standardize the drying time of the primary granules and of the granules containing polymer mixture. This study shows a stable water content between 2 and 5% after 24 hours of drying, both for the primary granules and for the granules containing the polymer mixture. This drying time, in accordance with the general characteristics of the granules, is maintained for the other assays. The water content of the granules is a parameter which can affect the stability of the granules. In effect, prolonged drying at 40°C can degrade the enoxaparin, and too high a water content may entail degradation of the polymers. The water can, indeed, play a role of plasticizer which modifies the physicochemical properties of the polymers, and thus reduces the stability of the drug form.

This manufacturing process of granules containing a polymer mixture of the active compound and a polymer (dispersion of Low molecular weight heparin (enoxaparin) in the cellulose polymer and subsequent mixing with different polymers for the formation of the final granule) constituted by two stages, permits reaching high manufacturing yields, with very little losses throughout the process. This result is very interesting within the framework of an industrial development, associated to not very high production and material costs.

_In vitro dissolution Kinetics of the granules containing polymer mixture_

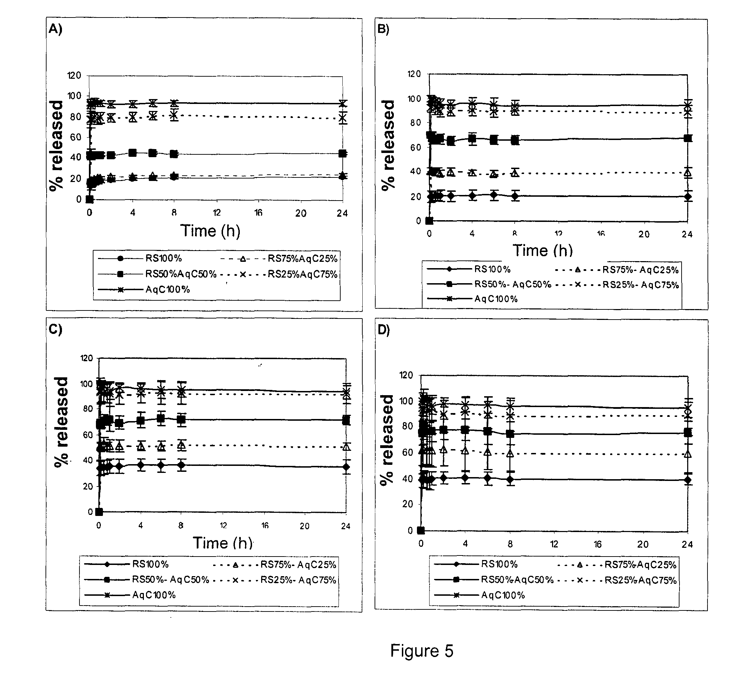

The results of the dissolution kinetics is expressed as percentage of enoxaparin released with respect to the initial quantity used during the preparation of the granules or in IU of enoxaparin released per gram of granules (Figure 5). The dissolution kinetics all have the same profile characterized by an important initial phase called "burst" wherein practically all the enoxaparin which can be released is released, and a second phase consisting of a plateau. We can also mention that the more Eudragit® RS 30 D that a formulation contains and the greater its percentage, the lower the number of IU of enoxaparin released. In contrast, the more ethyl cellulose a formulation contains, the greater the release of heparin.

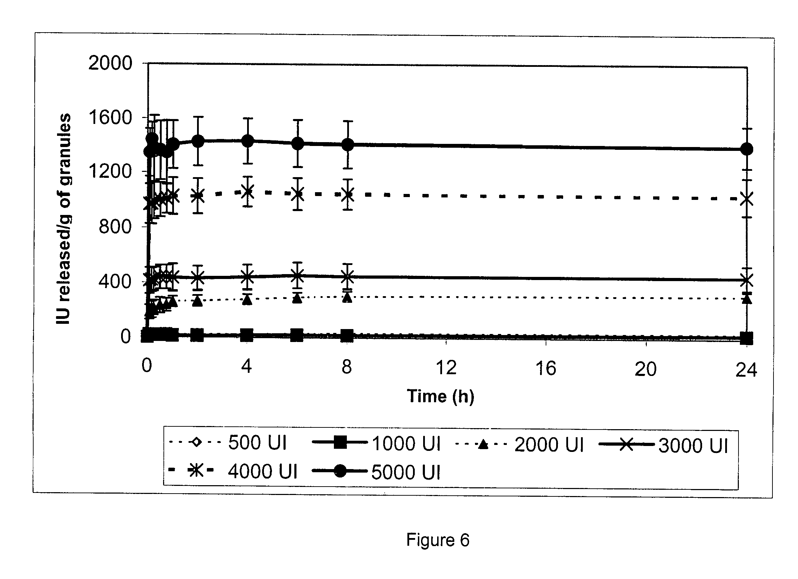

In vitro studies have permitted, in light of the dissolution kinetics, confirming the existence of electrostatic interactions between the quaternary ammonium groups of Eudragit® RS 30 D and the carboxyl and sulfate groups of enoxaparin. In effect, the more Eudragit® RS 30 D the granules contain, the less enoxaparin they release. In contrast, for the primary granules constituted by the mixture of microcrystalline cellulose and mixed with ethyl cellulose, the in vitro dissolution studies show a total release of the enoxaparin, which suggests an absence of interaction between enoxaparin and ethyl cellulose, a polymer without overall charge. Furthermore, for the same quantity of Eudragit® RS 30 D, the greater the increase in the quantity of enoxaparin used during the preparation of the granules, the greater quantity of enoxaparin released (figure 5). If we take as an example the primary granules of microcrystalline cellulose mixed with an aqueous dispersion which contains 100% Eudragit® RS 30 D (figure 6), no release of enoxaparin occurs in our experimental conditions when 500 and 1,000 units of anti-Xa have been used to prepare the granules, whilst there progressively appears a release for the granules which contain 2,000, 3,000, 4,000 or 5,000 IU of enoxaparin. We can therefore give the hypothesis that with 500 and 1,000 IU, the quantity of Eudragit® RS 30 D is sufficient to fix all the enoxaparin by electrostatic bonds which are difficult to break in the dissolution conditions of this assay. We can likewise suppose that the less heparin there is, the more the chains can be fixed to several points on the quaternary ammonium groups of Eudragit® RS 30 D, not favouring a release.

Furthermore, for the primary granules mixed with Eudragit® RS 30 D, associated or not to Aquacoat® ECD, the lower the initial quantity of enoxaparin used the lower the quantity of heparin released. This result is additionally confirmed by the fact that when 500 or 1,000 units of enoxaparin anti-Xa are used for the primary granules mixed with an aqueous suspension which contains 100% Eudragit® RS 30 D, the release of LMWH further decreases, until reaching zero (figure 6).

Whatever the quantity of enoxaparin in the granulates, the release profiles are all characterized by a more or less important immediate release, followed by a plateau. The initial release of enoxaparin may lead to the dissolution of the chains of enoxaparin not fixed on the Eudragit® RS 30 D, this quantity being more important the higher the quantity of enoxaparin in the granulates. Furthermore, the incomplete release of the heparin after 24 hours could be due to the fact that the interactions that bond the enoxaparin to the polymer are strong and, in any case, do not break in our experimental dissolution conditions. These results demonstrate that rising quantities of Eudragit RS 30 D present in the granulate matrix decrease the quantity of active compound released.

After the oral administration of granules mixed with aqueous suspensions containing 100% Eudragit® RS 30 D, 100% Aquacoat® ECD and a 50/50 mixture of Eudragit® RS 30 D/Aquacoat® ECD, an oral absorption of enoxaparin is observed, which demonstrates that there is no direct correlation between the in vitro and in vivo studies. In effect, the in vitro studies show that the primary granules of MCC only mixed with ethyl cellulose have the best release profile, whilst in vivo these same formulations have very low anti-Xa activities. In contrast, the primary granulates of MCC only mixed with Eudragit® RS 30 D have the greatest oral absorption profiles. This last result demonstrates the existence of possible interactions between the positive charges of the quaternary ammonium groups of Eudragit® RS and the negative charges of the intestinal mucosa, thus confirming the mucoadhesive properties of the polyacrylic derivatives (Jiao et al., 2002(c)). The adhesion of the granules on the digestive wall may create a concentration gradient which would facilitate the release of enoxaparin in the proximities of the intestinal mucosa. On the other hand, Eudragit® RS 30 D, polycationic polymer, may perhaps, such as chitosan, favour the opening of the tight bonds, and thus permit the passage of enoxaparin by paracellular route. The presence for its part of MCC on the surface would favour the release of sufficient quantities of active compound, demonstrating that the combined presence of both polymers on the surface balances the necessary relation between affinity for the absorption mucosa and the release of active compound.

These results allow the enoxaparin granules to occupy an interesting place among the group of other strategies which demonstrate the administration of heparins by oral route. These results show a strong potential, which have also been confirmed by the in vivo administration of formulations constituted by a primary granule formed by an aqueous dispersion of Low Molecular Weight Heparins on a MCC core which is subsequently coated by the coating in fluidized bed technique with an organic solution of a mixture of Eudragit RS PO and Polycaprolactone in a 50/50 proportion.

Eudragit® RS PO is a copolymer of trimethylammonioethyl methacrylate chloride which has an overall positive charge with a low concentration in quaternary ammonium groups (from 8 to 12%). This polymer is a non-biodegradable synthetic polymer. It is not absorbed or metabolized, for which reason it is eliminated intact. It is insoluble in water but soluble in organic solvents. It is presented in the form of dry powder.

## Example 6: In vivo studies of granules with Enoxaparin

### Measurement of anti-Xa activity

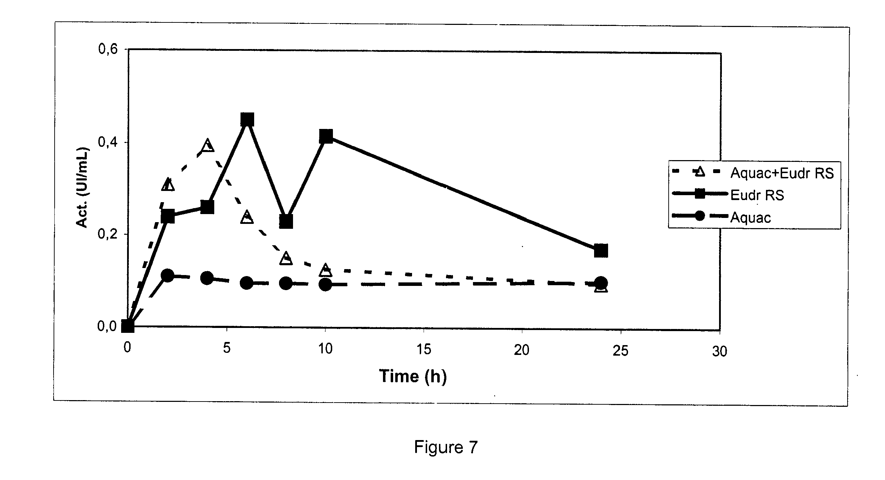

Figure 7 compares the plasma anti-Xa activities after oral administration to rabbits of enoxaparin granules (600 IU/kg) and after a subcutaneous injection of commercial enoxaparin solution (300 IU/kg). It can be observed that the primary granules of MCC only mixed with ethyl cellulose (Aquacoat) have a very low anti-Xa activity, indeed almost zero. In contrast, the primary granules .. of MCC only mixed with Eudragit® RS 30 D, which generate the most prolonged activity and the highest values of Cmax (Cmax = 0.45 $\pm$ 0.12 IU of anti-Xa/mL), correspond to a Tmax of 6 hours. The primary granules of MCC mixed with the aqueous suspension of Eudragit® RS and ethyl cellulose in 50/50 proportion lead to a slightly lower Cmax (Cmax = 0.4 $\pm$ 0.12 IU of anti-Xa/mL) corresponding to a Tmax of 4 hours.

### Relative bioavailabilities

After calculating the areas under the curve using the trapezoidal method, the relative bioavailabilities calculated 8 hours after oral administration of the granules have given the following values: 6.1% for the primary granules of MCC only mixed with ethyl cellulose, 17.8% for the primary granules of MCC mixed with Eudragit® RS, and 17.3% for the primary granules of MCC mixed with the Eudragit® RS/ethyl cellulose 50/50 mixture.

Whatever the quantity of enoxaparin in the granulates, the release profiles are all characterized by a more or less important immediate release, followed by a plateau. The initial release of enoxaparin may lead to the dissolution of the chains of enoxaparin not fixed on the Eudragit® RS 30 D, this quantity being more important the higher the quantity of enoxaparin in the granules. Furthermore, the incomplete release of the heparin after 24 hours could be due to the fact that the interactions that bond the enoxaparin to the polymer are strong and, in any case, do not break in our experimental dissolution conditions.

After the oral administration of primary granules of MCC mixed with aqueous suspensions containing 100% Eudragit® RS 30 D, 100% Aquacoai® ECD and a 50/50 mixture of Eudragit® RS 30 D/Aquacoat® ECD, an oral absorption of enoxaparin is observed, which demonstrates that there is no direct correlation between the in vitro and in vivo studies. Indeed, the in vitro studies show that the primary granules of MCC only mixed with ethyl cellulose have the best release profile, whilst in vivo these same formulations have very low anti-Xa activities. In contrast, the primary granules of MCC only mixed with Eudragit® RS 30 D have the greatest oral absorption profiles. This last result demonstrates the existence of possible interactions between the positive charges of the quaternary ammonium groups of Eudragit® RS and the negative charges of the intestinal mucosa, thus confirming the mucoadhesive properties of the polyacrylic derivatives, whilst the cellulose derivative would permit the release of sufficiently high levels of active compound. The adhesion of granulates on the digestive wall may create a concentration gradient which would facilitate the release of enoxaparin in the proximities of the intestinal mucosa. On the other hand, Eudragit® RS 30 D, polycationic polymer, such as chitosan, probably favours the opening of the tight bonds, and thus permits the passage of enoxaparin by paracellular route.

These first results permit the enoxaparin granulates to occupy and interesting place among the group of other strategies which demonstrate the administration of heparins by oral route. These results show a strong potential of the formulations prepared in the present invention.

Example 7: **Preparation of granules containing Tinzaparin**

Tinzaparin is a low molecular weight heparin whose antithrombotic and anticoagulant activities are dissociated. It is characterized by higher anti-Xa activity than anti-IIa activity, the proportion between both activities being 1.8.

The form used for our experiments is an aqueous solution of INNOHEP® dosed at 18,000 IU of anti-Xa / 0.9 mL and commercially designed for the subcutaneous route.

The polymers used for the creation of the polymeric matrix are:

Cellulose:

In this example its solid form dissolved in acetone at different concentrations has been used.

Poly-ε-caprolactone (PCL):

PCL is a semicrystalline aliphatic polyester with a molecular mass of 42000 g/mol. It is a biodegradable polymer, for which hydrolysis gives rise to the formation of water soluble monomers which are eliminated in the urine (Chen DR et al. Polycaprolactone microparticles and their biodegradation. Polymer degradation and stability, 2000, 52: 117-126). PCL is insoluble in water and soluble in organic solvents such as acetone. The PCL has been obtained from Sigma-Aldrich.

The processes used for granulation have been the following: The manufacturing of the granulates is carried out in two stages. The first stage, called primary granulation, consists of mixing the aqueous solution of tinzaparin (5,000 IU of anti-Xa) with 1 g of microcrystalline cellulose (MCC). After drying the mass, the agglomerates are forced to pass through a metal sieve with mesh opening of 600 μm. The primary granulates thus produced are then dried for 5 minutes in the oven at 40°C

The second stage, of regranulation, consists of adding a solution of Eudragit RS and PCL in equivalent proportions in acetone (100 mg/ml of each of the two polymers) to the granules. The addition of different quantities of this polymer mixture to the primary granules of cellulose and tinzaparin has been studied. Thus, by varying the volume of organic solution added to the previously produced primary granules, granules have been obtained with quantities of the Eudragit RS and PCL mixture in quantities of 200, 400, 600 and 800 mg per gram of cellulose. The combination is dried in the oven at 40°C for 30 minutes. Once dry, the agglomerates are forced to pass through the metal sieve with a mesh opening equal to 600 μm. The granules produced are dried in the oven at 40°C for 5 minutes.

The proportions of the different polymers thus produced are shown in the following table:

| Formulation | % Cellulose | % Eudragit RS | % PCL |
|---|---|---|---|
| 1 | 83.33 | 8.33 | 8.33 |
| 2 | 71.43 | 14.28 | 14.28 |
| 3 | 62.50 | 18.75 | 18.75 |
| 4 | 55.55 | 22.22 | 22.22 |

The "virgin" granules, without heparin, have been prepared in the same manner, substituting the heparin solution for water.

In an alternative regranulation process, a solution of Eudragit RS in acetone (200 mg/ml) is added to the primary granules. The addition of different quantities of this Eudragit RS solution to the primary granules of cellulose and tinzaparin has been studied. Thus, by varying the volume of organic solution added to the previously produced primary granules, granules have been obtained with quantities of Eudragit RS in quantities of 200, 400, 600 and 800 mg per gram of cellulose. The combination is dried in the oven at 40°C for 30 minutes. Once dry, the agglomerates are forced to pass through the metal screen with a mesh opening equal to 600 μm. The granules produced are dried in the oven at 40°C for 5 minutes.

The proportions of the different polymers thus produced are shown in the following table:

| Formulation | % Cellulose | % Eudragit RS |
|---|---|---|
| 5 | 83.33 | 16.66 |
| 6 | 71.43 | 28.57 |

(continued)

| Formulation | % Cellulose | % Eudragit RS |
|---|---|---|
| 7 | 62.50 | 37.50 |
| 8 | 55.55 | 44.44 |

The "virgin" granules, without heparin, have been prepared in the same manner, substituting the heparin solution for water.

Characterization of the granules produced:

*Morphology and size*

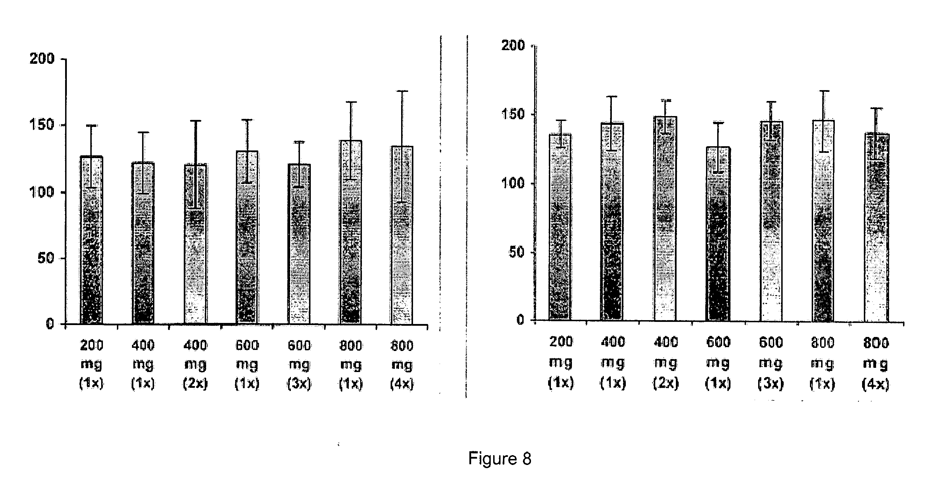

The particle size of the granules has been determined by the laser diffraction technique in a Malvern Mastersizer apparatus. The primary granules are resuspended in pH 7.4 phosphate buffer. During the measurement, the suspension is maintained by stirring the measuring basin.
The average size of the granules produced has been in all cases between 100 and 150 $\mu$m as depicted in figure 8.

*In vitro release kinetics*

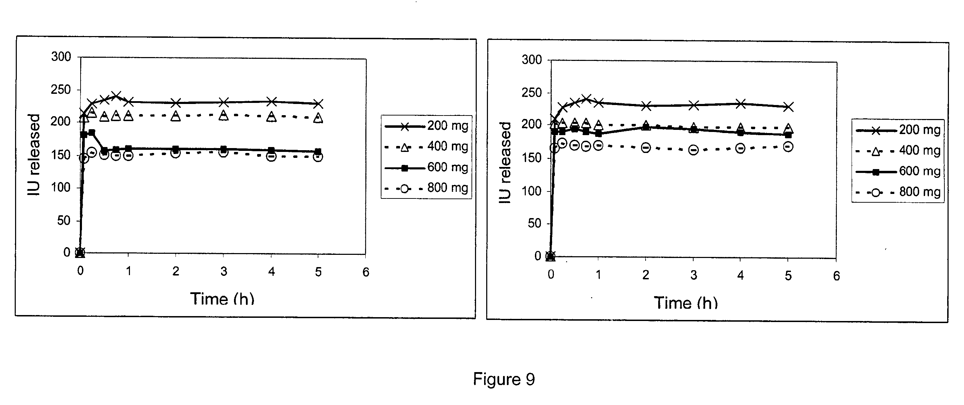

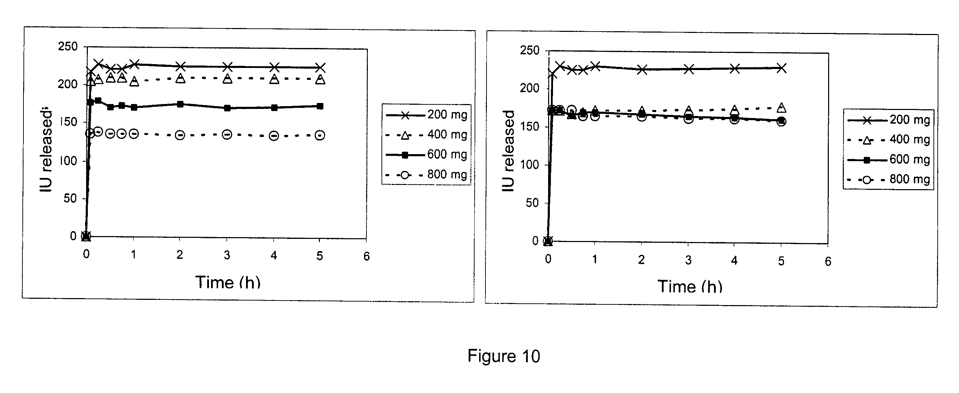

The objective of this assay is to determine the quantity of tinzaparin released by the granules over time. This *in vitro* release should be performed in defined conditions called "sink", i.e. those wherein the maximum concentration of active compound which can be released in a release medium is less than 30% of the saturation concentration, so that the concentration gradient can be considered non-limiting.
The release assays have been carried out in a water bath thermostatted at 37˚C, suspending with gentle magnetic stirring (300 rpm) 50 mg of each batch of tinzaparin granules in 20 mL of phosphate buffer (PBS, 0.011 M and NaCl, 0.15 M) at pH 7.4 containing 0.1 % Tween 80.
1 mL samples were taken with the automatic pipette after 5, 10, 15, 30, 45 minutes, and after 1, 2, 3, 4, 5, 6, 7, 8 and 24 hours. The volumes collected were substituted by 1 mL of fresh buffer in order to maintain a constant volume in the flasks. These aliquots were then filtered with a MILLIPORE filter with 0.22 $\mu$m porosity.
The quantities of tinzaparin released were evaluated by nephelometry, and then the curves which represent the release kinetics of tinzaparin in accordance with time are established. The release kinetics were performed in triplicate for each of the samples (n=3). The results are shown in figures 9 and 10.
The granules produced have a two-phase release profile, with a rapid initial release followed by a sustained release. As can be seen in figures 9 and 10, a greater quantity of polymers added to the primary core reduces the release of the tinzaparin contained therein. This fact also occurs both in the primary granules mixed with Eudragit RS as well as in those mixed with the Eudragit RS and PCL solution, and in both it is certainly related to the increase in interaction between tinzaparin and Eudragit RS.

**Example 8: In vivo studies of granules with Tinzaparin**

Measurement of the anti-Xa activity

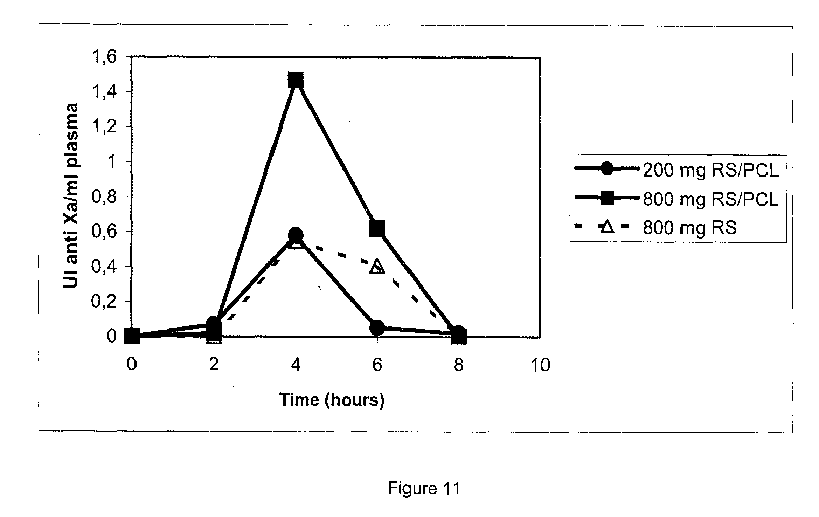

Figure 11 compares plasma anti-Xa activity after the oral administration to rabbits of granules with tinzaparin (600 IU/kg). It has been observed that the primary granules of MCC mixed with 800 mg of Eudragit RS and PCL in equivalent quantities generate a much higher tinzaparin absorption than that obtained with the primary granules mixed with 200 mg of the Eudragit RS and PCL solution. In light of these results, the increase in the percentage of Eudragit RS polycation with respect to the presence of polymers with a negative or neutral charge (8.33% in the formulation wherein 200mg are added and more than 22% in the formulation wherein 800 mg are added) is clearly demonstrated. This increase in absorption is shown through a higher Cmax value (1.58 compared with 0.57 IU anti-Xa/ml) as well as through an increase in the area under the curve and the bioavailability (F=28.26 compared with F=15.46). As can be clearly observed, the increase in bioavailability is not related to a greater quantity of active compound released from the formulations, since the formulations constituted by primary granules of MCC which are mixed with 200 mg of the Eudragit RS and PCL polymeric solution release more tinzaparin and at a greater rate than the formulations that are mixed with 800 mg of the Eudragit RS and PCL solution.
The in vivo assay demonstrates that tinzaparin, normally not absorbable after its oral administration in the form of solution, is absorbed in significantly high quantities when it is administered in the form of granules when they have a suitable composition. The increasing quantities of Eudragit RS favour a greater interaction of the granule with the intestinal wall and mainly with the negatively charged mucosa that covers it, whilst the presence of the biodegradable polyester, PCL, favours the in vivo release of tinzaparin in suitable quantities. This hypothesis is supported by the fact that the primary granules mixed with 800 mg of Eudragit RS and PCL have a greater absorption profile than the primary granules of MCC which are mixed with 800 mg of Eudragit RS.

## Example 9: Preparation of granules containing Bemiparin

Bemiparin is a low molecular weight heparin whose antithrombotic and anticoagulant activities are dissociated. It is characterized by higher anti-Xa activity than anti-IIa activity, the proportion between both activities being between 2 and 4.
The form used for our experiments is an aqueous Bemiparin solution starting from lyophilized Bemiparin (ROVI laboratories) dosed at 5,000 IU of anti-Xa / mL.
The manufacturing of the granules is carried out in two stages. The first stage, called primary granulation, consists of mixing the aqueous Bemiparin solution (5,000 IU of anti-Xa in 1 ml) with 1 g of microcrystalline cellulose (MCC). After drying the mass, the agglomerates are forced to pass through a metal sieve with mesh opening of 600 $\mu$m. The primary granules thus produced are then dried for 5 minutes in the oven at 40˚C
The second stage, of regranulation, consists of adding an aqueous dispersion of Eudragit RS (Eudragit RS 30 D) to the primary granules. The combination is dried in the oven at 40˚C for 30 minutes. Once dry, the agglomerates are forced to pass through the metal sieve with a mesh opening equal to 600 $\mu$m. The granules produced are dried in the oven at 40˚C for 5 minutes.
The proportions of the different polymers thus produced are shown in the following table:

| Formulation | % Cellulose | % Eudragit RS |
|---|---|---|
| 1 | 55.55 | 44.44 |

Characterization of the granules produced:

*Morphology and size*

The particle size of the granules has been determined by laser diffraction technique in a Malvern Mastersizer apparatus. The granules are first resuspended in pH 7.4 phosphate buffer. During the measurement, the suspension is maintained by stirring the measuring basin.
The average size of the granules produced is of around 150 $\mu$m.

In vivo studies of granules with Bemiparin

Measurement of the anti-Xa activity

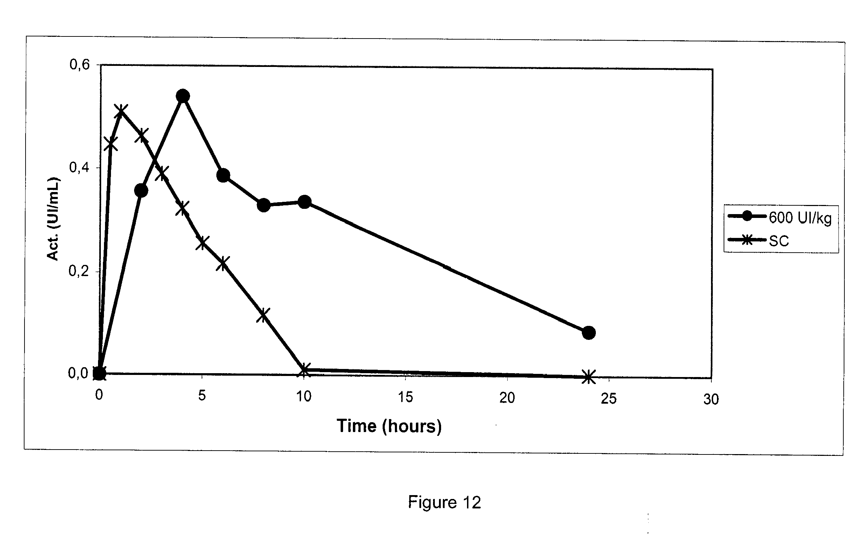

Figure 12 compares the plasma anti-Xa activities after the oral administration to rabbits of granules with bemiparin at a dose of 600 IU/kg. Similarly to that produced with enoxaparin and tinzaparin granules, the granules containing Bemiparin give rise to significantly high plasma levels. The bioavailability relative to the subcutaneous administration of Bemiparin dissolved in water at a dose of 150 IU/kg was 60.94%, and the Cmax of 0.54 IU/ml in plasma after 4 hours.

## Example 10: Preparation of pellets containing Enoxaparin

The form used for the experiments is an aqueous solution of LOVENOX® dosed at 10,000 IU of anti-Xa / mL and commercially designed for the subcutaneous route.
The following table shows the composition of the pellets in percentage with respect to the final solid mass. Likewise, the quantity of water used in the preparation of the pellets is included in the table by way of illustration:

| Eudragit RS 30D[a] | Acetyltriethylcitrate plasticizer | MCC | Lactose | Enoxaparin sodium [a,b] |
|---|---|---|---|---|
| 20.76% | 0.4% | 55.23% | 21.10% | 2.51% |

Comments:

(a) Calculated as mass of the solid
(b) LOVENOX®: Enoxaparin sodium at a concentration of 10000 IU/ml

- Eudragit RS 30D dispersion- 69.20 g, of which 20.76 g correspond to the dry mass of the polymer and 48.44 g correspond to the liquid phase
- Enoxaparin solution (LOVENOX) - 24.03 g, of which 2.51 g correspond to dry enoxaparin and 21.52 g correspond to water

First, the MCC, the lactose and the plasticizer are mixed for 2 minutes in a mixer (Tefal Kaleo, France). Then the Eudragit RS dispersion at a flow of 10 ml/min and the aqueous solution of enoxaparin are gradually added, subjecting the combination to homogenization for 1 minute. The mass produced is extruded through 0.6mm pores (thickness of the extruded mass is 1 mm) and subsequently it is spheronized for 15 minutes at 640 rpm. The resulting pellets are dried in a ventilated oven at 40°C during 48 hours.

The granules thus produced contain 25.1 mg of Enoxaparin per gram of pellets.

Despite the fact that all components of the pellet are homogeneously distributed throughout the formulation, the proportion of polymers that form the matrix and, therefore, are on the surface is significantly high (55.23% of MCC and 20.76% of Eudragit RS). Their non-presence in greater quantity is probably the cause that the bioavailability obtained is not at the level of the granules, as it will become apparent below.

Characterization of the pellets produced:

*Morphology and size*

The particle size of the pellets produced has been determined by optical microscope. The procedure permits preparing pellets with diameters between 0.5 and 1.2 mm. For the in vitro and in vivo assays, the mass of pellets is sieved to separate them into two portions with sizes comprised between 0.5-0.8 mm and 0.8-1.2 mm.

*In vitro release kinetics*

The objective of this assay is to determine the quantity of enoxaparin released by the pellets over time. This *in vitro* release should be performed under defined conditions called "sink", i.e. those wherein the maximum concentration of active compound which can be released in a release medium is lower than 30% of the saturation concentration, so that the concentration gradient can be considered non-limiting.

The release assays have been carried out in a bath thermostatted at 37°C, suspending with gentle magnetic stirring (300 rpm) 50 mg of each batch of enoxaparin pellets in 20 mL of phosphate buffer (PBS, 0.011 M and NaCl, 0.15 M) at pH 7,4, containing 0.1% of Tween 80.

1 mL samples have been taken with the automatic pipette after 5, 15, and 30 minutes, and after 1, 2, 4 and 24 hours. The volumes collected are substituted by 1 mL of new buffer in order to maintain a constant volume in the flasks. These aliquots are then filtered with a MILLIPORE filter with 0.22 $\mu$m porosity.

The quantities of enoxaparin released are evaluated by nephelometry, and then the curves which represent the release kinetics of enoxaparin as a function of time are established. The release kinetics have been performed in triplicate for each one of the samples (n=3).

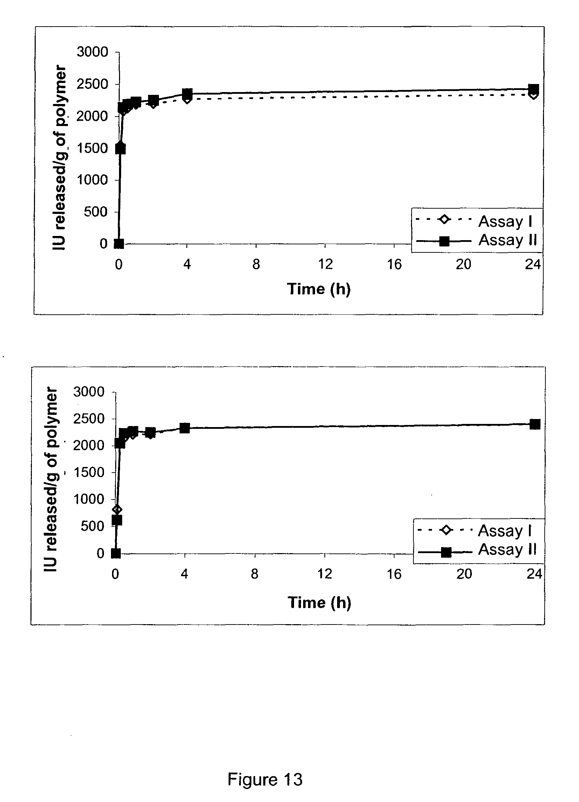

The pellets produced have a two-phase release profile, with a rapid initial release followed by a sustained release. As can be seen in figure 13, no relevant differences are observed in relation to the release rate of the active compound in accordance with the pellet size, in all cases quickly releasing the enoxaparin.

**Example 11: In vivo studies of pellets with Enoxaparin**

Measurement of the anti-Xa activity

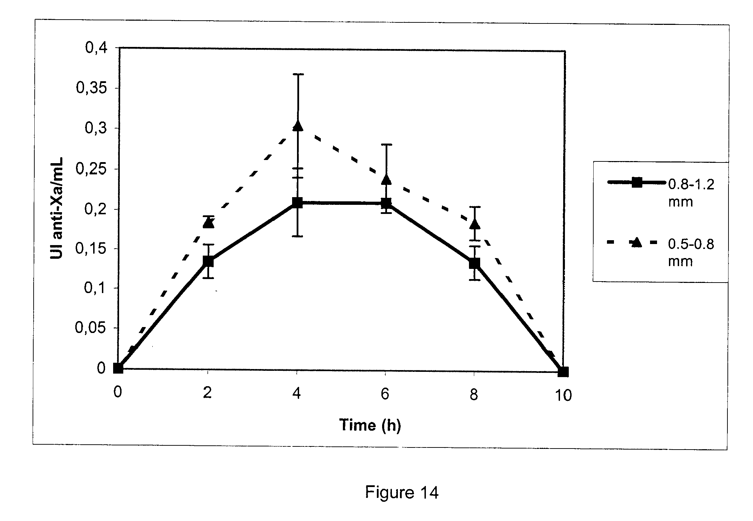

Figure 14 compares the plasma anti-Xa activities after the oral administration to rabbits of pellets with enoxaparin (600 IU/kg). As it can be observed in the figure, the oral administration to NZW rabbits of the pellets, with sizes between 0.5-0.8 mm and 0.8-1.2 mm provide significantly high absorptions, with plasma levels which are maintained over a period of 10 hours. The Cmax obtained with pellets of size between 0.5 and 0.8 mm is 0.305 IU/ml after 4 hours, whilst that obtained with pellets of size between 0.8 and 1.2 mm is 0.21 IU/ml after 4-6 hours. The values of absolute bioavailability obtained are respectively 13.7 % for pellets of size between 0.5-0.8 mm and 10.4% for pellets of size between 0.8-1.2 mm. Despite the fact that the pellets of smaller size provide greater bioavailability than the larger ones, it is surprising to obtain bioavailabilities of this magnitude with such large particles.

This in vivo assay demonstrates that the appropriate use of polymers in the preparation of formulations permits achieving the absorption of enoxaparin in significant quantities after its administration by oral route. The combined effect of a sufficient adhesion to the mucosal wall and an efficient release of the active compound provided by Eudragit RS and Microcrystalline cellulose is sufficiently significant to permit active compounds poorly permeable to the absorption barrier to pass through it, despite the large size of the pellets which may exceed the millimetre in diameter. This observation is surprising in light of the information that may be found today in the state of the art, which continually stresses the need to reduce the formulation size to produce more efficient dispersions with a greater contact time with the absorption mucosa. It is also obvious for a person skilled in the art that the formulations included in the examples herein have further undeniable advantages over the production of microparticles and nanoparticles, since they are produced by processes commonly used in the pharmaceutical industry, they have less variability in interbatch production and are easily scalable to industrial batches, unlike what happens with other formulations.

## Example 12: Formation of coated granules containing Bemiparin

The manufacturing of the granules is carried out in two stages. The first stage, called core preparation, consists of preparing primary bemiparin granules with microcrystalline cellulose (MCC). A high-speed Zanchetta Rotolab® granulator/mixer has been used to prepare the granules.

**Composition:**

| Avicel pH 101 | 100 g | 95.24% |
|---|---|---|
| Bemiparin | 5 g | 4.76% |
| Water milli-Q | q.s. | |

**Preparation procedure:**

1. Dissolve the Bemiparin in 50 mL of milli-Q water and place it in the dropping funnel.

2. Weigh the exact quantity of avicel and place it in the granulator container.

3. Add the Bemiparin solution to the avicel in movement due to the drive blade

4. Check the degree of wetting of the avicel and the formation of granules.

5. Add the necessary quantity of milli-Q water until the granules are formed.

6. Remove the granules from the equipment and sieve them through a sieve with a mesh opening of 1 mm.

7. Dry in oven at 37°C for 2 hours and then at ambient temperature.

**Equipment conditions:**

| Speed of the impeller (drive blade) | | 250-300 rpm |
|---|---|---|
| Chopper | | Off |
| Heating jacket | Off | |

The second stage, of coating, consists of adding a polymer mixture to the primary granules, either dissolved or in suspension, by spraying of the primary cores in fluidized bed (Mini Glatt® with microkit accessory). The primary granules are then coated directly with different proportions of each of the polymers.

**Composition:**

| Coating solution: | Eudragit RS PO | 2.5 g |
|---|---|---|
| | PCL | 2.5 g |
| | Acetone | 100 mL |

To perform the coating of the previously produced primary granules, we introduce 30 grams thereof in the granulator to coat them with the aforementioned coating solution wherein both polymers are in a 1:1 ratio. Granules have been prepared with different coating percentages (2.5, 4 and 8% w/w in relation to the initial mass of primary granules introduced). The same concentration of solids has been used in all of them, 5% by weight with respect to the acetone volume, and the greater or lesser degree of coating is achieved by varying the solution volume used to coat the primary granule.

**Preparation procedure:**

1. Place the inner core (30 g) in the product filling tubulator of the fluidized bed

2. Fill the hose with the coating solution until the spray nozzle.

3. Switch on the equipment and adjust the process parameters

4. Perform the coating and remove the end product from the tubulator.

**Process parameters:**

| Temperature (˚C) | Pump index | Air pressure of the process (bars) | Spray air pressure (bars) | Air temperature of the process (˚C) |
|---|---|---|---|---|
| Ambient | 1.0 | 0.08-0.15 | 0.50 | 25 |

Once the spraying of the polymeric solution has concluded, the process air is maintained during 20 seconds for the coating to dry.

Characterization of the granules produced:

*Morphology and size*

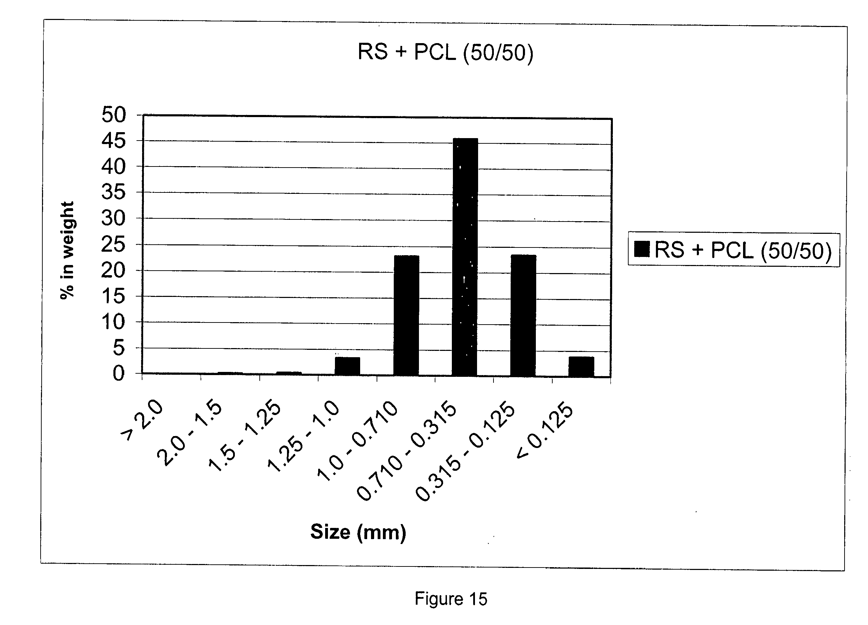

The granulates coated with the Eudragit RS and PCL matrix, the coating representing a gain of 4% over the weight of the primary MCC and Bemiparin granule, have been observed under the optical microscope in these conditions and after having been dispersed in a 4% PVA solution. The size has been determined by separation by sieving, using sieves with mesh openings of 2, 1.5, 1.25, 1, 0.71, 0.315 and 0.125 mm. The result is expressed in percentage of the total mass measured which the different fractions obtained represent. The distribution of sizes obtained can be observed in figure 15.

As can be observed in the figure, 72.82% of total mass of the granulates have a particle size over 0.315mm, the main fraction being that which has a size between 0.71 and 0.315mm, which constitutes 45.85% of the total mass of the granulate.

*In vitro release kinetics*

The release assays have been performed in sink conditions in a 6-vessel dissolution apparatus with water bath thermostatted at 37˚C. 166mg of granules produced (with 4% coating) have been introduced in a hard number 1 gelatine capsule and the capsules have been introduced in spinner baskets which are immersed in the dissolution medium (400 ml of pH 6.8 phosphate buffer). The spinner basket rotation rate has been maintained at 100 rpm.

1.5 mL samples have been taken with the automatic pipette after 30 minutes, and after 1, 1.5, 2, 4, 6 and 24 hours. The volumes collected are substituted by 1.5 mL of new buffer in order to maintain a constant volume in the vessels. These aliquots are then filtered with a MILLIPORE filter with 0.22 $\mu$m porosity.

The quantities of bemiparin released are evaluated by nephelometry, and then the curves which represent the release kinetics of bemiparin as a function of time are established. The release kinetics have been performed in duplicate for each one of the samples (n=2).

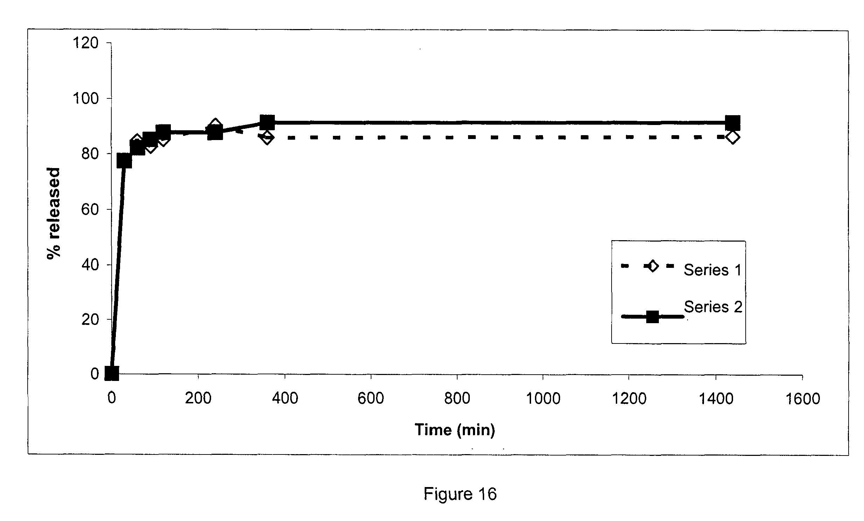

As can be observed in figure 16, the release profile is two-phase, similar to that produced with the granules prepared by mixing. In the two measurements made the percentages released after an hour exceed 80% of the total incorporated. In this case it can be observed that the quantity of Eudragit RS and PCL is very low in the granule with respect to that incorporated in the formulations produced by mixing. Nevertheless, the polymeric coating containing the Eudragit RS and PCL matrix is capable of releasing the active compound in a similar way to that produced with the previous formulations. Despite the fact that certain retention of Bemiparin in the granule is observed, probably due to its interaction with the cationic polymer, the quantity of active compound is sufficient for it to be released in significantly high quantities.

**Example 13: In vivo studies of granules with Bemiparin**

Measurement of the anti-Xa activity

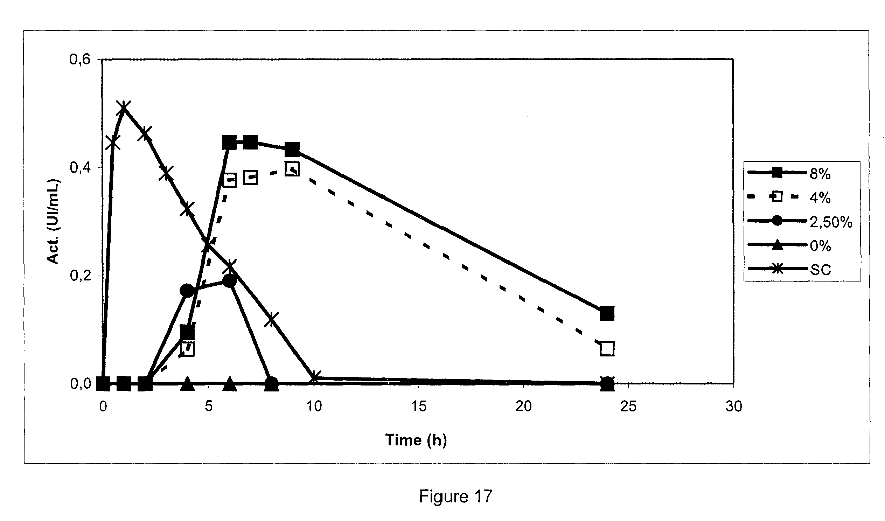

Figure 17 compares the plasma anti-Xa activities after the oral administration to rabbits of hard gelatine capsules (with enteric coating of Eudragit L) containing Bemiparin granules produced according to example 12 (600 IU/kg). As can be observed in the figure, the polymeric coating plays an extremely important role in the biological activity of the administered formulations, since the oral administration of bemiparin in uncoated MCC granules does not cause the appearance of plasma levels of the active compound. Likewise, a clear influence is observed of the coating thickness in the biological activity of the granules produced. Despite the fact that the coating of the primary granule with a 2.5% weight gain of the mixture of Eudragit RS and PCL gives rise to the presence of detectable plasma levels, probably the quantity of coating added is not sufficient to completely coat primary granulate. However, additions of the polymer mixture which represent a weight gain of 4 and 8% provide high absorptions, with plasma levels which are maintained for a 24-hour period. The Values of Cmax and the relative bioavailabilities obtained for each one of these formulations are reflected in the following table:

| % coating | Cmax | F(%) relative to 150 IU/kg sc |
|---|---|---|
| 0 | 0 | 0 |
| 2.5 | 0.90 | 5.58 |
| 4 | 0.3978 | 39.59 |
| 8 | 0.447 | 47.66 |

It is important to highlight that in these examples Eudragit RS and the PCL are found in equivalent quantities on the surface. The combined effect of a sufficient adhesion to the mucosal wall and an efficient release of the active compound provided by Eudragit RS and PLC is sufficiently important to permit active compounds poorly permeable to the absorption barrier to pass through it.

For the purposes of clarifying the results obtained and demonstrating the activity achieved with the suitable polymeric material, the incorporation of excipients commonly used in granulation procedures has been avoided intentionally (both for the core and for the coating) but it is also obvious for a person skilled in the art that this preparation procedure can also be performed using any of the excipients that may be required to optimize the granulation process.

Additionally, anti-Xa activity has been studied in the plasma of NZW rabbits after the oral administration of 600 IU/kg of bemiparin granules prepared according to the procedure described in example 18 with a 5% coating on non-enteric gelatine capsules.

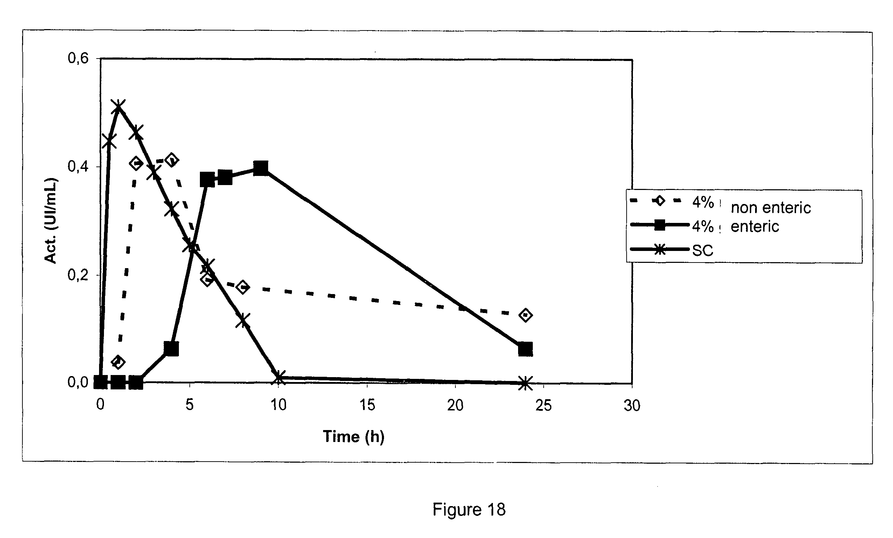

As can be seen in figure 18, the enteric coating modifies the kinetic profile obtained only in relation to the appearance time of the anti-Xa activity plasma levels, but neither the Cmax obtained nor the bioavailability significantly vary with the use of enteric coatings in the gelatine capsules. Therefore, it is sufficiently demonstrated that the absorption of bemiparin obtained is not related to the release of formulations in the intestinal tract, but the formulations enable the absorption of bemiparin after a single oral administration. The delay observed in the appearance of plasma levels in the case of the enteric capsules is directly related to the time necessary for the capsule to pass through the digestive cavity and to start to disintegrate in a suitable pH environment in the duodenum.

**Example 14: Formation of coated granules containing Bemiparin**

The manufacturing of the granules is carried out in two stages. The first stage, called core preparation, consists of preparing primary bemiparin granules with microcrystalline cellulose (MCC). A high-speed Zanchetta Rotolab® granulator/mixer has been used to prepare the granules.

**Composition:**

| | | |
|---|---|---|
| Avicel pH 101 | 100 g | 95.24% |
| Bemiparin | 5 g | 4.76% |
| Water milli-Q | q.s. | |

**Preparation procedure:**

1. Dissolve the Bemiparin in 50 mL of milli-Q water and place it in the dropping funnel.

2. Weigh the exact quantity of avicel and place it in the granulator container.

3. Add the Bemiparin solution to the avicel in movement due to the drive blade

4. Check the degree of wetting of the avicel and the formation of granulate.

5. Add the necessary quantity of milli-Q water until the granulate is formed.

6. Remove the granulate from the equipment and sieve it through a sieve with a mesh opening of 1 mm.

7. Dry in oven at 37˚C for 2 hours and then at ambient temperature.

**Equipment conditions:**

| | |
|---|---|
| Speed of the impeller (drive blade) | 250-300 rpm |
| Chopper | Off |
| Heating jacket | Off |

The second stage, of coating, consists of adding a polymer mixture to the primary granulates, either dissolved or in suspension, by spraying of the primary cores in fluidized bed (Mini Glatt® with microkit accessory). The primary granulates are then coated directly with different proportions of each of the polymers.

**Composition:**

| | | |
|---|---|---|
| Coating solution: | Eudragit RS PO | 4.5 g |

(continued)

| | |
|---|---|
| PCL | 0.5 g |
| Acetone | 100 mL |

To perform the coating of the previously produced primary granules, 30 grams thereof are introduced in the granulator to coat them with the aforementioned coating solution wherein the Eudragit RS PO: PCL ratio is 9:1. Granules have been prepared with a coating percentage of 5% w/w in relation to the initial mass of the primary granules introduced.

**Preparation procedure:**

1. Place the inner core (30 g) in the product filling tubulator of the fluidized bed

2. Fill the hose with the coating solution until the spray nozzle.

3. Switch the equipment on and adjust the process parameters

4. Perform the coating and remove the end product from the tubulator.

**Process parameters:**

| Temperature (˚C) | Pump index | Air pressure of the process (bars) | Spray air pressure (bars) | Air temperature of the process (˚C) |
|---|---|---|---|---|
| Ambient | 1.0 | 0.08-0.15 | 0.50 | 25 |

Once the spraying of the polymeric solution has concluded, the process air is maintained during 20 seconds for the coating to dry.

Characterization of the granules produced:

*Morphology and size*

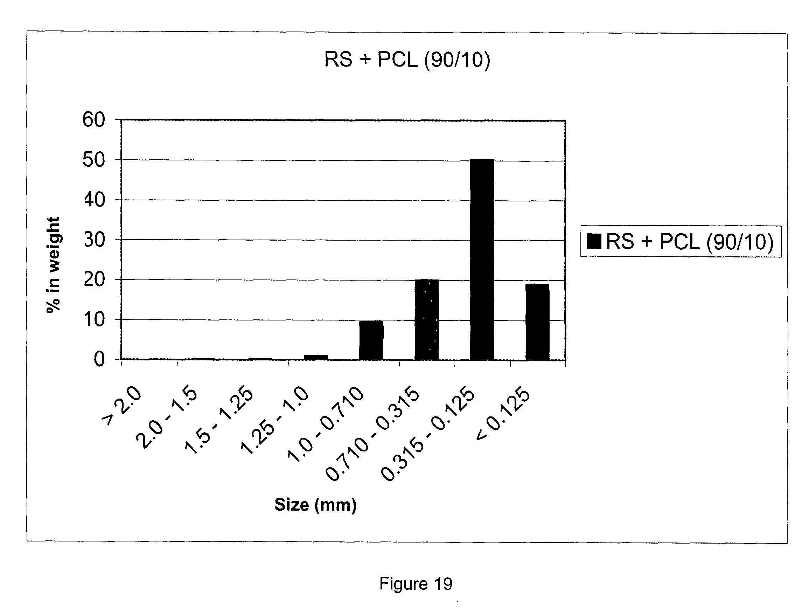

The granulate coated with the Eudragit RS and PCL matrix (in 9:1 ratio), the coating representing a gain of 4% over the weight of the primary MCC and Bemiparin granule, have been observed under the optical microscope in these conditions and after having been dispersed in a 4% PVA solution. The size has been determined by separation by sieving, using sieves with mesh openings of 2, 1.5, 1.25, 1, 0.71, 0.315 and 0.125 mm. The result is expressed in percentage of the total mass measured which the different fractions obtained represent. The distribution of sizes obtained can be observed in figure 19.

As can be observed in the figure, the resulting particle size is smaller when a 1:1 ratio is used between the Eudragit RS and the PCL present in the coating. A 50.30% of the total mass of the granules has a particle size between 0.315mm and 0.125mm.

**Example 15: In vivo studies of granules with Bemiparin of example 14**

Measurement of the anti-Xa activity

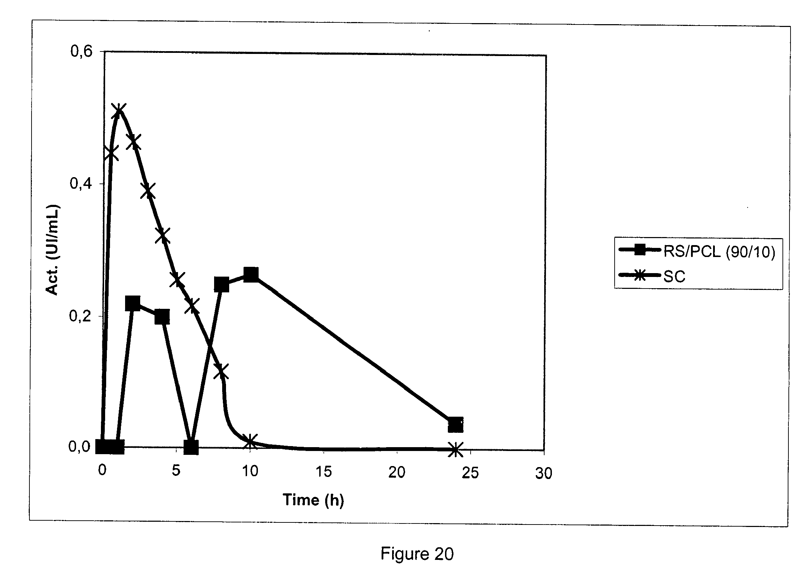

Figure 20 compares the plasma anti-Xa activities after the oral administration to rabbits of hard gelatine capsules (with enteric coating of Eudragit L) containing Bemiparin granules produced according to example 14 (600 IU/kg). As can be observed in the figure, the increase of the proportion of Eudragit RS on the surface up to a ratio of 9:1 in relation to the PCL gives rise to clearly lower levels of plasma activity than those obtained with the administration of granules which have equivalent surface quantities of Eudragit RS and PCL. However, significantly high absorptions can still be reached in this ratio. The greater presence of Eudragit RS and the greater interaction produced with the Bemiparin contained in the granule conditions the release of the active compound and, therefore, the absorption thereof, despite the greater bioadhesiveness this polymer gives to the formulation. Values of Cmax and the relative bioavailabilities obtained are reflected in the following table:

| % coating | Cmax | F(%) relative to 150 IU/kg sc |
|---|---|---|
| 5 | 0.265 | 25.37 |

## Example 16: Formation of coated granules containing Bemiparin and having an intermediate coating layer

The formulations included in this example aim to demonstrate the possibility of adding subcoats with polymers between the primary core and the outer coating with the Eudragit RS and PCL polymer mixture to avoid contact between the active compound and the polymers that enable the absorption thereof without the activity of the polymeric coating as promoter of Bemiparin absorption varying significantly. The manufacturing of granulates is carried out in three stages. The first stage, called core preparation, consists of preparing primary bemiparin granules with microcrystalline cellulose (MCC). A high-speed Zanchetta Rotolab® granulator/mixer has been used to prepare the granules.

**Composition:**

| Avicel pH 101 | 100 g | 95.24% |
|---|---|---|
| Bemiparin | 5 g | 4.76% |
| Water milli-Q | q.s. | |

**Preparation procedure:**

1. Dissolve the Bemiparin in 50 mL of milli-Q water and place it in the dropping funnel.

2. Weigh the exact quantity of avicel and place it in the granulator container.

3. Add the Bemiparin solution to the avicel in movement due to the drive blade

4. Check the degree of wetting of the avicel and the formation of granulate.

5. Add the necessary quantity of milli-Q water until the granulate is formed.

6. Remove the granulate from the equipment and sieve them through a sieve with a mesh opening of 1 mm.

7. Dry in oven at 37°C for 2 hours and then at ambient temperature.

**Equipment conditions:**

| Speed of the impeller (drive blade) | 250-300 rpm |
|---|---|
| Chopper | Off |
| Heating jacket | Off |

The second stage, of subcoat, consists of adding a polymer mixture to the primary granules, either dissolved or in suspension, by spraying of the primary cores in fluidized bed (Mini Glatt® with microkit accessory). The primary granules are then coated directly with the polymer until obtaining a weight gain of 4%.

**Composition:**

Coating solution:  Eudragit L    5 g
                   Acetone      100 mL

To perform the coating of the previously produced primary granules, 30 grams thereof are introduced in the granulator to coat them with the aforementioned subcoat solution.

**Preparation procedure:**

1. Place the inner core (30 g) in the product filling tubulator of the fluidized bed

2. Fill the hose with the coating solution up to the spray nozzle.

3. Switch the equipment on and adjust the process parameters

4. Perform the coating and remove the end product from the tubulator.

**Process parameters:**

| Temperature (˚C) | Pump index | Air pressure of the process (bars) | Spray air pressure (bars) | Air temperature of the process (˚C) |
|---|---|---|---|---|
| Ambient | 1.0 | 0.08-0.15 | 0.50 | 25 |

Once the spraying of the polymeric solution has concluded, the process air is maintained during 20 seconds for the coating to dry.

The third stage, of coating, consists of adding to the primary granules a polymeric coating containing Eudragit RS PO and PCL in a proportion of 1:1, by spraying of the cores produced in the second step in a fluidized bed (Mini Glatt® with microkit accessory). The granulates with subcoat are then directly coated with the polymer mixture until obtaining a weight gain of 4%. The coating procedure and parameters are identical to those of the subcoating stages.

Characterization of the granules produced:

*Morphology and size*

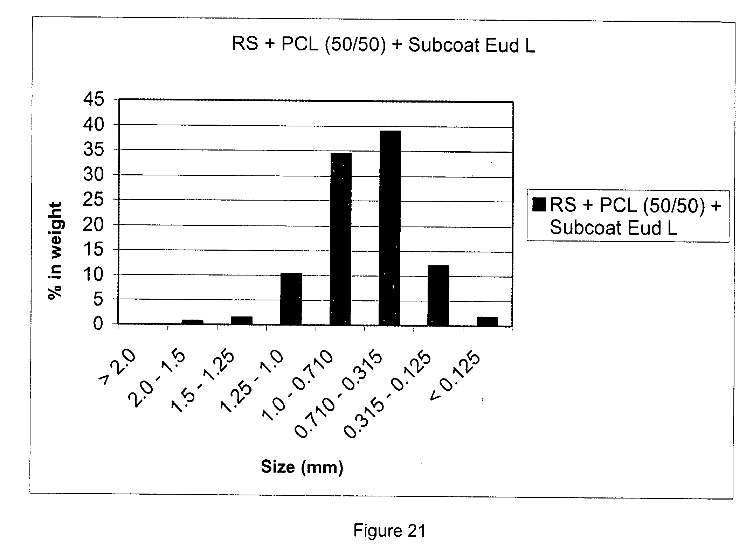

The granules coated with the Eudragit RS and PCL matrix and with a subcoat of Eudragit L on the primary MCC and Bemiparin granule have been observed under the optical microscope in these conditions and after having been dispersed in a 4% PVA solution. The size has been determined by separation by sieving, using sieves with mesh openings of 2, 1.5, 1.25, 1, 0.71, 0.315 and 0.125 mm. The result is expressed in percentage of the total mass measured which the different fractions obtained represent. The distribution of sizes obtained can be observed in figure 21.

As can be observed in figure 21, the addition of a subcoat increases the particle size of the granules produced, the fraction which has a particle size over 0.315mm being 86.10% of the total mass of the granules. The main fraction is that which represents particles between 0.71 and 0.315 mm, with 39.01% of the total.

**Example 17: In vivo studies of coated granules containing Bemiparin and having an intermediate coating layer**

Measurement of the anti-Xa activity

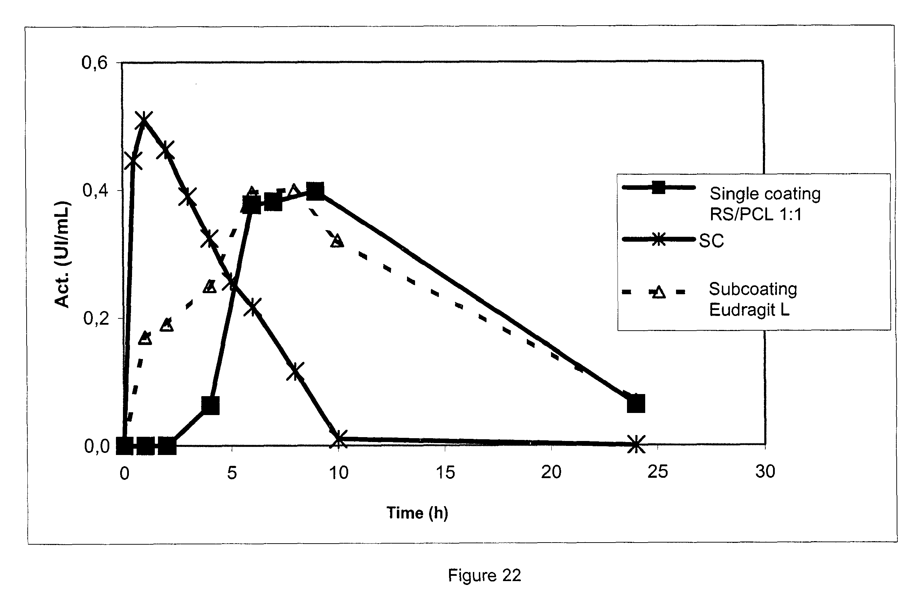

Figure 22 compares the plasma anti-Xa activities after the oral administration to rabbits of non-enteric hard gelatine capsules containing Bemiparin granules produced according to example 16 and their comparison with bemiparin granules only containing the outer coating of Eudragit RS and PCL in a proportion of 1:1 according to example 12 (600 IU/kg). As is evident in the figure, the application of a subcoat in the granule structure maintains the Bemiparin absorption levels obtained without a subcoat. In this case it is evident that the presence of active compound on the surface is not structurally necessary for the vehicle to achieve the absorption of the active compound, as there is a physical separation between the active compound and the polymeric mixture on the surface. Once the formulation is administered, the granule is hydrated and the Bemiparin starts to diffuse outwards, and it is the suitable ratio between the interaction of the polymeric coating with the digestive tract wall and the release of the active compound which achieves that Bemiparin is absorbed in sufficiently high quantities. Likewise, it is evident that the absorption of Bemiparin achieved in the granule using the same compositions on the surface is equivalent and independent of the internal structure of the particulate vehicle provided that the active compound is quickly released.

Values of Cmax and the relative bioavailabilities obtained for each one of these formulations are reflected in the following table:

| % coating | Cmax | F(%) relative to 150 IU/kg sc |
|---|---|---|
| 4 (only outer) | 0.3978 | 39.59 |
| 4 (subcoat)+4 (outer) | 0.400 | 45.63 |

Despite the fact that the subcoat has been prepared by spraying a polymer solution in acetone, it can be easily deduced by any person skilled in the art that similar results can be obtained using any other polymer solvent or by spraying an aqueous suspension thereof that the company (Degussa, Germany) markets under the name Eudragit L 30D

For the purposes of clarifying the results obtained and demonstrating the activity achieved with the suitable polymeric mixture, the incorporation of excipients commonly used in granulation procedures has been avoided intentionally (both for the core and for the coating) but it is also obvious for a person skilled in the art that this preparation procedure can also be performed using any of the excipients that may be required to optimize the granulation process.

### Example 18: Formation of coated granules containing Bemiparin

The manufacturing of the granules is carried out in two stages. The first stage, called core preparation, consists of preparing primary bemiparin granules with microcrystalline cellulose (MCC). A high-speed Zanchetta Rotolab® granulator/mixer has been used to prepare the granules.

**Composition:**

| | | |
|---|---|---|
| Avicel pH 101 | 90 g | 85.71% |
| Bemiparin | 15 g | 14.29% |
| Water milli-Q | q.s. | |

**Preparation procedure:**

1. Dissolve the Bemiparin in 50 mL of milli-Q water and place it in the dropping funnel.

2. Weigh the exact quantity of avicel and place it in the granulator container.

3. Add the Bemiparin solution to the avicel in movement due to the drive blade

4. Check the degree of wetting of the avicel and the formation of the granulate.

5. Add the necessary quantity of milli-Q water until the granulatee is formed.

6. Remove the granulate from the equipment and sieve them through a sieve with a mesh opening of 1 mm.

7. Dry in oven at 37˚C for 2 hours and then at ambient temperature.

**Equipment conditions:**

| | |
|---|---|
| Speed of the impeller (drive blade) | 250-300 rpm |
| Chopper | Off |
| Heating jacket | Off |

The second stage, of coating, consists of adding a polymer mixture to the primary granules, either dissolved or in suspension, by spraying of the primary cores in fluidized bed (Mini Glatt® with microkit accessory). The primary granulates are then coated directly with different proportions of each of the polymers.

**Composition:**

| Coating solution: | Eudragit RS PO | 2.5 g |
|---|---|---|
| | Pluronic F68 | 2.5 g |
| | Acetone | 100 mL |

To perform the coating of the previously produced primary granules, we introduce 30 grams thereof in the granulator to coat them in the aforementioned coating solution wherein both polymers are in a 1:1 ratio. Granules have been prepared with a coating percentage of 5% w/w in relation to the initial mass of primary granules introduced.

**Preparation procedure:**

1. Place the inner core (30 g) in the product filling tubulator of the fluidized bed

2. Fill the hose with the coating solution up to the spray nozzle.

3. Switch on the equipment and adjust the process parameters

4. Perform the coating and remove the end product from the tubulator.

**Process parameters:**

| Temperature (˚C) | Pump index | Air pressure of the process (bars) | Spray air pressure (bars) | Air temperature of the process (˚C) |
|---|---|---|---|---|
| Ambient | 1.0 | 0.08-0.15 | 0.50 | 25 |

Once the spraying of the polymeric solution has concluded, the process air is maintained during 20 seconds for the coating to dry.

Characterization of the granules produced:

*Morphology and size*

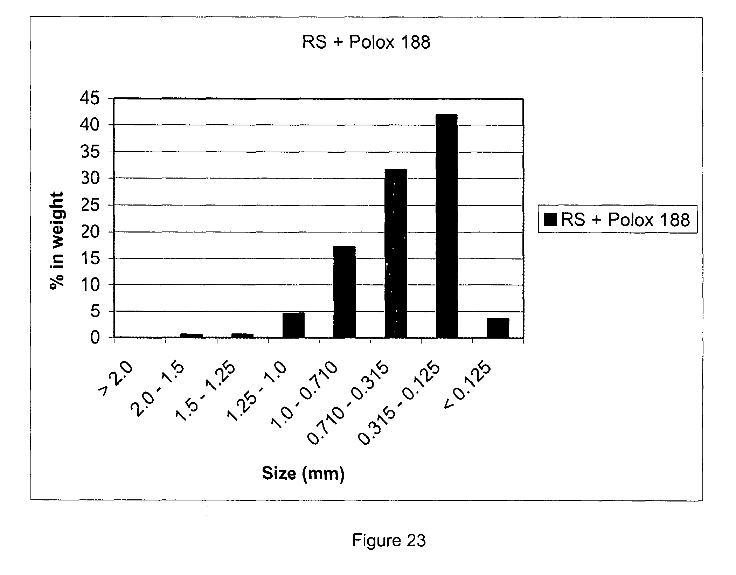

The granules coated with the Eudragit RS and Pluronic F68 matrix (in 1:1 ratio), the coating representing a gain of 4% over the weight of the primary MCC and Bemiparin granule, have been observed under the optical microscope in these conditions and after having been dispersed in a 4% PVA solution. The size has been determined by separation by sieving, using sieves with mesh openings of 2, 1.5, 1.25, 1, 0.71, 0.315 and 0.125 mm. The result is expressed in percentage of the total mass measured which the different fractions obtained represent. The distribution of sizes obtained can be observed in figure 23.

As can be observed in the figure, 96.44% of the total mass of the granulate have a particle size over 0.125mm.

*In vitro release kinetics*

The release assays have been performed in sink conditions in a 6-vessel dissolution apparatus with water bath thermostatted at 37˚C. 166mg of granules produced (with 4% coating) have been introduced in a hard number 1 gelatine capsule and the capsules have been introduced in spinner baskets which are immersed in the dissolution medium (400 ml of pH 6.8 phosphate buffer). The spinner basket rotation rate has been maintained at 100 rpm.

1.5 mL samples have been taken with the automatic pipette after 30 minutes, and after 1, 1.5, 2, 4, 6 and 24 hours. The volumes collected are substituted by 1.5 mL of fresh buffer in order to maintain a constant volume within the vessels. These aliquots are then filtered with a MILLIPORE filter of 0.22 $\mu$m porosity.

The quantities of bemiparin released are evaluated by nephelometry, and then the curves which represent the release kinetics of bemiparin as a function of time are established. The release kinetics have been performed in duplicate for each one of the samples (n=2).

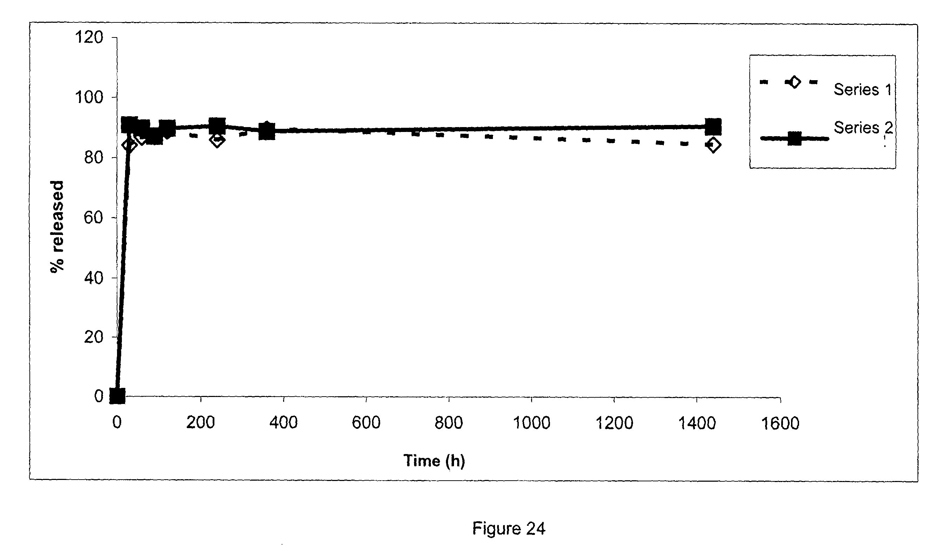

Figure 24 shows that the release profile of these formulations is two-phase. In this case, the initial release represents more than 80% in all the measurements made. It can be clearly observed that Poloxamer 188, which is a polymer which shows avidity for water, facilitates the hydration of the granule and the release of the bemiparin contained therein.

## Example 19: In vivo studies of granules with Bemiparin of example 18

Measurement of the anti-Xa activity

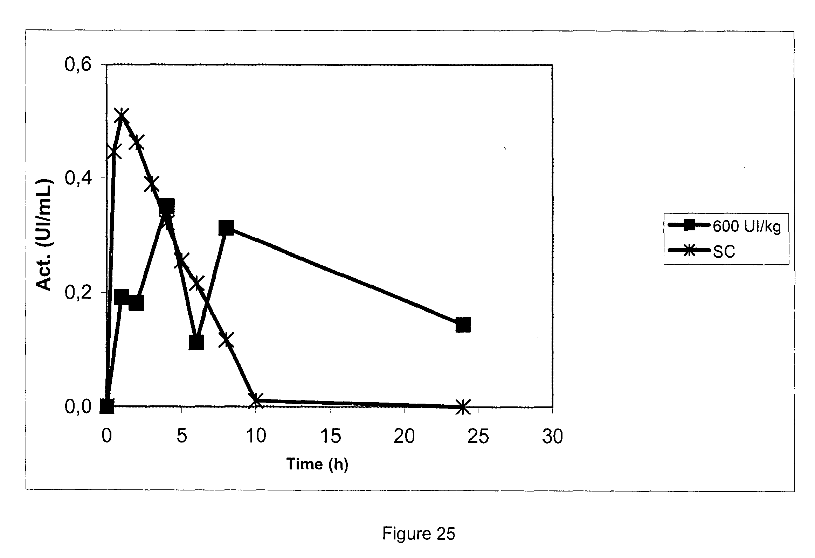

Figure 25 compares the plasma anti-Xa activities after the oral administration to rabbits of hard gelatine capsules (with enteric coating of Eudragit L) containing Bemiparin granules produced according to example 18 (600 IU/kg). As can be observed in the figure, the administration of bemiparin granules which present in the coating a polymeric matrix constituted by Eudragit RS and Pluronic F68 in 1:1 proportion gives rise to significantly high levels of plasma activity. Values of Cmax and the relative bioavailabilities obtained are reflected in the following table:

| % coating | Cmax | F(%) relative to 150 IU/kg sc |
|---|---|---|
| 5 | 0.352 | 41.20 |

By way of conclusion, it can be highlighted that the appropriate use of polymers in the preparation of formulations permits achieving the absorption of poorly permeable active compounds in significant quantities after their administration by oral route. The combined effect of a sufficient adhesion to the mucosal wall and an efficient release of the active compound provided by Eudragit RS and the polymers that combine with it in suitable proportions is sufficiently important to permit active compounds, poorly permeable to the absorption barrier to pass through it, despite the large size of the formulations presented by way of example which may exceed one millimetre in diameter. This observation is surprising in light of the information that may be found today in the state of the art, which continually stresses the need to reduce the formulation size to produce more efficient dispersions with a greater contact time with the absorption mucosa. It is also obvious for a person skilled in the art that the formulations included in the examples herein have further undeniable advantages over the production of microparticles and nanoparticles, since they are produced by processes commonly used in the pharmaceutical industry, they have less variability in the interbatch production and are easily scalable to industrial batches, unlike what happens with the other formulations.

## Example 20: Bemiparin suspension in a dissolution of Eudragit RS PO and polycaprolactone

The suspension was obtained in three steps. In a first step, 9,25 mg of Eudragit RS PO and 9,25 mg of PCL were dissolved in 1,85 mL of acetone. Subsequently, a dissolution of Bemiparin in water (18,5 mg of Bemiparin in 200 $\mu$L of water) was prepared. Finally, the aqueous dissolution was added to the dissolution of Eudragit RS PO and PCL in acetone, and the whole mass was subjected to magnetic stirring for 5 minutes.

The suspension of Bemiparin in acetone (without polymers) was performed as follows: a dissolution of 18,5 mg of

Bemiparin in 200 μL of water was added to 1,85 mL of acetone. The whole mass was subjected to magnetic stirring for 5 minutes.

**Example 21: In vivo studies of the Bemiparin suspension of example 20**

Measurement of the anti-Xa activity

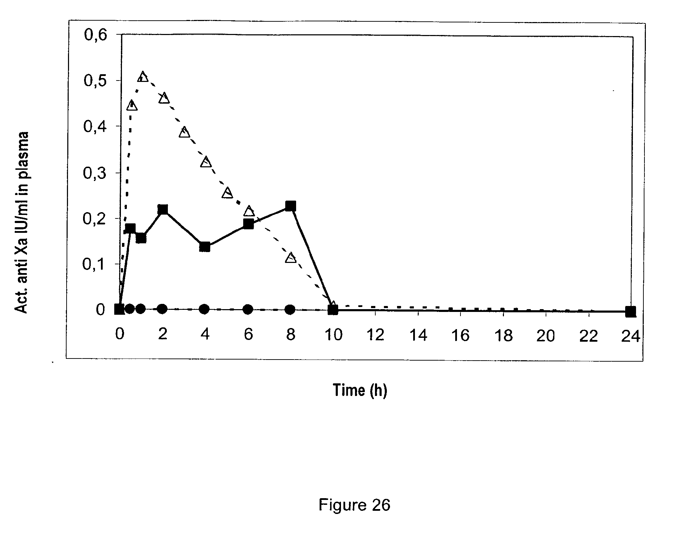

Figure 26 shows the plasma anti-Xa activities after the oral adminsitration to rabbits of suspensions obtained according to example 20 (600 IU/kg). As it can be observed in the figure, the administration of Bemiparin suspension in a dissolution of Eudragit RS PO and PCL in acetone (in a ratio of polymers of 1:1) gives rise to significantly high levels of plasma anti-Xa activity. Values of Cmax and the relative bioavailability obtained are reflected in the following table:

| Cmax | F(%) relative to 150 UI/kg sc |
|---|---|
| 0,218 | 25,00 |

Likewise, it can be observed that the administration of the Bemiparin suspension in acetone without either Eudragit RS PO or PCL does not give rise to the apparition of levels of plasma anti-Xa activity in rabbits. This shows that the acetone by itself, does not excert any efect on the absorption of Bemiparin upon its oral administration.

The difusion of acetone toward the gastrointestinal fluids produces the precipitation of Eudragit RS PO and PCL, conforming a solid matrix which encompasses Bemiparin. The active principle diffuses from the matrix and upon its absorption, it passes to systemic circulation.

**Claims**

1.   Pharmaceutical form containing at least an active compound and a polymeric matrix, **characterised in that** said polymeric matrix comprises at least one polymer of cationic nature.

2.   Pharmaceutical form according to claim 1, wherein the polymeric matrix forms part of the surface of the said form.

3.   Pharmaceutical form according to claims 1 or 2, wherein the polymeric matrix is in contact with the active principle.

4.   Pharmaceutical form according to any of claims 1 to 3, wherein the polymeric matrix comprises at least one polymer of cationic nature and at least another polymer of anionic or neutral nature.

5.   Pharmaceutical form according to any of claims 1 to 4, wherein the release of the active compound is of at least 50% in a time lower than or equal to 60 minutes.

6.   Pharmaceutical form according to any of claims 1 to 5, having a particle size of at least 0,1mm.

7.   Pharmaceutical form according to any of claims 1 to 6, wherein the polymeric matrix comprises additionally compounds selected from plasticizers, glidants, absorption enhancers, humectants, surfactants, colouring agents, and dispersants.

8.   Pharmaceutical form according to any of claims 1 to 7, wherein said form is a non extended release form.

9.   Pharmaceutical form according to any of claims 1 to 8, wherein the active compound is a poorly permeable hydrophilic molecule containing groups with negative charges at pH greater than or equal to 4.

10.  Pharmaceutical form according to any of claims 1 to 9, wherein the active compound is selected from the group consisting of: antibiotics, anti-inflammatories, anti-infectious agents, antiparasitics, hormones, substances with immunological activity, vaccines, immunomodulators, immunosuppressants, cytostatics, diuretics, agents with activity in the digestive system, agents with activity in the circulatory system, agents with activity in the respiratory system, human growth hormone, recombinant growth hormone, bovine growth hormone, growth-hormone releasing hormone, interferons, analgesics, agents with activity in the central nervous system, erythropoietin, somatostatin, gonadotropin-releasing hormone, follicle-stimulating hormone, oxytocin, vasopressin, parathyroid hormone, adrenocorticotropin, gonadotropin-releasing hormone, thrombopoietin, calcitonin, interferons, interleukins, insulins, unfractionated heparin, low molecular weight heparin, ultra low molecular weight heparins, heparinoids, dermatan, glucosamines, chondroitins, auricular natriuretic factor, monoclonal antibodies, protease inhibitors, filgrastim, prostaglandins (PGE2 and PGI2), cyclosporin, cromolyn sodium, cromoglycate and its salts, vasopressin, vancomycin, neomycin, desferrioxamine, antimicrobial agents, antifungals, cytostatics, immunomodulators, vitamins, antivirals, antigens, ribonucleic acid, deoxyribonucleic acid, oligonucleotides, CPG sequences, plasmids, active compounds of protein nature, active compounds of polysaccharide nature, glucocerebrosidase, and derivatives and combinations thereof.

11. Pharmaceutical form according to claim 10, wherein the active compound is selected from the group consisting of: insulins, unfractionated heparin, low molecular weight heparin, ultra low molecular weight heparins and heparinoids.

12. Pharmaceutical form according to claim 11, wherein the active compound is low molecular weight heparin.

13. Pharmaceutical form according to any of claims 4 to 12, wherein at least one polymer of anionic or neutral nature and at least one polymer of cationic nature are present in a proportion of at least 10 % each in relation to the weight of the polymeric matrix.

14. Pharmaceutical form according to any of claims 1 to 13, wherein the polymer of cationic nature in the polymeric matrix is selected from polymers and copolymers derived from acrylic and methacrylic acids, and natural polymers.

15. Pharmaceutical form according to any of claims 4 to 13, wherein the polymer of anionic or neutral nature in the polymeric matrix is selected from the group consisting of: polyesters, polycaprolactones, polymers and copolymers derived from acrylic acid, polyethylene oxides, polypropylene oxides, polyethylene and polypropylene oxide copolymers, polyanhydrides, polyamides, polyurethanes, polycarbonates, polyacetals, polyorthoesters, polycyanacrilates, polydioxanones, poly ($\alpha$-hydroxy acids), poly ($\beta$-hydroxy acids) polyphosphazenes, natural polymers; mixtures, copolymers and terpolymers thereof.

16. Pharmaceutical form according to claim 15, wherein among the polymers of anionic or neutral nature the polyesters are selected from the-group consisting of: lactic acid polymers, glycolic acid polymers, lactic and glycolic acid copolymers; the polycaprolactone is poly-$\varepsilon$-caprolactone; the acrylic acid derived polymers are selected from the group of polymethylmethacrylates; and the natural polymers are selected from the group of cellulose derivatives formed by microcrystalline cellulose, hydroxypropylmethylcellulose and ethyl cellulose; mixtures, copolymers and terpolymers thereof.

17. Pharmaceutical form according to any of claims 4 to 13, 15 and 16, wherein the polymers of anionic and neutral nature in the polymeric matrix are biodegradable.

18. Pharmaceutical form according to any of claims 4 to 16, wherein the polymeric matrix comprises polyesters and natural biodegradable polymers as polymers of anionic or neutral nature and methacrylic acid derivatives with quaternary ammonium groups as polymers of cationic nature.

19. Pharmaceutical form according to any of claims 4 to 16, wherein the polymeric matrix comprises poly-$\varepsilon$-caprolactone as a polymer of anionic or neutral nature and a polymer derived from methacrylic acid with quaternary ammonium groups as a polymer of cationic nature.

20. Pharmaceutical form according to claim 19, wherein the polymer derived from methacrylic acid with quaternary ammonium groups is a trimethylammonioethyl methacrylate chloride copolymer.

21. Pharmaceutical form according to any of claims 4 to 15, wherein the polymeric matrix comprises a polyethylene and polypropylene oxide copolymer as polymer of neutral nature and a polymer derived from methacrylic acid with quaternary ammonium groups as polymer of cationic nature.

22. Pharmaceutical form according to any of claims 1 to 21, **characterized in that** it is a granule.

23. Pharmaceutical form according to claim 22, wherein the granule comprises a core and a polymeric matrix, wherein said core comprises at least an active compound and said polymeric matrix comprises at least one polymer of cationic nature.

24. Pharmaceutical form according to claim 23, wherein the polymeric matrix further comprises at least one polymer of anionic or neutral nature.

25. Pharmaceutical form according to claims 23 or 24, wherein the polymeric matrix forms part of the core.

26. Pharmaceutical form according to claims 23 or 24, wherein the polymeric matrix forms a coating layer on or over the core.

27. Pharmaceutical form according to claim 26, which further contains an intermediate layer between the core and the coating layer.

28. Pharmaceutical form according to claims 26 or 27, wherein the core comprises an inert seed and a coating layer comprising the active compound.

29. Pharmaceutical form according to claim 28, which further comprises an intermediate layer between the inert seed and the layer comprising the active compound.

30. Pharmaceutical form according to any of claims 1 to 21, **characterized in that** it is a pellet.

31. Pharmaceutical form according to claim 30 wherein the pellet comprises at least one active compound and a polymeric matrix, wherein said polymeric matrix comprises at least one polymer of cationic nature.

32. Pharmaceutical form according to claim 31 wherein the polymeric matrix further comprises at least one polymer of anionic or neutral nature.

33. Process for the preparation of a pharmaceutical form according to any of claims 1 to 32 that comprises combining at least an active compound and at least one polymer of cationic nature in conditions that a polymeric matrix is formed.

34. Process according to claim 33, which comprises the step of subjecting a composition comprising at least one active compound to a granulation process to produce a core.

35. Process according to claims 34, wherein the composition further comprises at least one polymer of cationic nature.

36. Process according to claim 35, wherein the composition further comprises at least one polymer of anionic o neutral nature.

37. Process according to a claim 35, further comprising coating the core with a composition comprising at least one polymer of cationic nature.

38. Process according to a claim 37, wherein the composition further comprises at least one polymer of anionic o neutral nature.

39. Process according to claim 33 comprising the steps of :

   a) coating an inert seed with a composition comprising at lest one active compound to obtain a core, and
   b) coating the core prepared in a) with a composition comprising at least a polymer of cationic nature.

40. Process according to claim 39, which further comprises in step a) coating the inert seed with a composition, to form an intermediate coating layer before performing the coating with a composition comprising at least one active compound.

41. Process according to claims 39 or 40, wherein in step b) the composition further comprises at least one polymer of anionic o neutral nature.

42. Process according to any of claims 39 to 41" wherein in step b) a spray method is used.

43. Process according to claims 37 or 38, or 39 to 42, which further comprises coating the core with a composition to form an intermediate coating layer.

**44.** Process according to claim 33 that comprises the steps of:

   a) mixing at least one active compound with at least one polymer of cationic nature, and
   b) extruding the homogenized mixture.

**45.** Process according to claim 44 wherein in step a) the active compound is further mixed with at least one polymer of anionic or neutral nature.

**46.** Pharmaceutical formulation comprising a pharmaceutical form as defined in claims 1 to 32.

**47.** Pharmaceutical formulation according to claim 46, wherein said formulation is a non extended release formulation

**48.** Pharmaceutical formulation according to claims 46 or 47, **characterized in that** it takes the form of a tablet, a capsule, a granule, a pellet, a lyophilisate, a serum, a microparticle, an oily suspension, a liposome, a micelle, a self-emulsifying system and other dispersed systems.

**49.** Pharmaceutical formulation according to any of claims claims 46 to 48, **characterized in that** it is for administration by oral route.

**50.** Use of the pharmaceutical form as defined in claims 1 to 32, for the preparation of a pharmaceutical formulation.

**51.** Use according to claim 50 wherein said formulation is a non extended release formulation.

**52.** Use of the pharmaceutical form as defined in claims 1 to 32, or a pharmaceutical formulation as defined in claims 46 to 49, for the manufacture of a medicament applicable to mucosas selected from oropharyngeal mucosa, gastrointestinal mucosa, pulmonary mucosa, nasal mucosa and vaginal mucosa.

**53.** Pharmaceutical form as defined in claims 1 to 32 or a pharmaceutical formulation as defined in claims 46 to 49, for use as a medicament applicable to mucosas selected from oropharyngeal mucosa, gastrointestinal mucosa, pulmonary mucosa, nasal mucosa and vaginal mucosa.

Diagram 1

Diagram 2

2 mL of enoxaparin solution

1g of MCC

Drying 2h30 at 40°C
Sieving
Drying 24h at 40°C

**Primary Granules**

aqueous suspension of 400 mg of polymers

Primary granules

Mixing
Drying 30min at 40°C
Sieving
Drying 24h at 40°C

**Granules**

EP 2 060 253 A1

Figure 1

Figure 2

Figure 3

Figure 4

Figure 5

Figure 6

Figure 7

Figure 8

Figure 9

Figure 10

Figure 11

Figure 12

EP 2 060 253 A1

Figure 13

Figure 14

Figure 15

EP 2 060 253 A1

Figure 16

EP 2 060 253 A1

Figure 17

EP 2 060 253 A1

Figure 18

Figure 19

EP 2 060 253 A1

Figure 20

EP 2 060 253 A1

Figure 21

EP 2 060 253 A1

Figure 22

Figure 23

EP 2 060 253 A1

Figure 24

Figure 25

Figure 26

European Patent Office

# EUROPEAN SEARCH REPORT

Application Number

EP 07 38 0319

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/03960 A (MAINCENT PHILIPPE [FR]; UBRICH NATHALIE [FR]; VIGNERON CLAUDE [FR]) 17 January 2002 (2002-01-17) * examples 1,10; tables 1,2 * | 1-53 | INV. A61K9/16 A61K9/50 A61K31/727 A61K38/28 A61K9/58 |
| X | HOFFART V. ET AL.: "Oral bioavailability of a low molecular weight heparin using a polymeric delivery system" JOURNAL OF CONTROLLED RELEASE, vol. 113, 2006, pages 38-42, XP002475915 * the whole document * | 1-53 | |
| X | WO 2005/032703 A (DEBIO RECH PHARMA SA [CH]; CURDY CATHERINE [CH]; DUCREY BERTRAND [CH];) 14 April 2005 (2005-04-14) * example 19 * | 1-53 | |

TECHNICAL FIELDS SEARCHED (IPC)

A61K

The present search report has been drawn up for all claims

| Place of search | Date of completion of the search | Examiner |
|---|---|---|
| Munich | 10 April 2008 | Giménez Miralles, J |

EPO FORM 1503 03.82 (P04C01)

## EP 2 060 253 A1

ANNEX TO THE EUROPEAN SEARCH REPORT
ON EUROPEAN PATENT APPLICATION NO.

EP 07 38 0319

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2008

| Patent document cited in search report | | Publication date | Patent family member(s) | Publication date |
|---|---|---|---|---|
| WO 0203960 | A | 17-01-2002 | AU 1874502 A | 21-01-2002 |
| | | | BR 0112080 A | 06-05-2003 |
| | | | CA 2415414 A1 | 17-01-2002 |
| | | | CN 1440277 A | 03-09-2003 |
| | | | EP 1313453 A1 | 28-05-2003 |
| | | | FR 2811227 A1 | 11-01-2002 |
| | | | JP 2004502720 T | 29-01-2004 |
| | | | MX PA03000193 A | 13-09-2004 |
| | | | US 2005013866 A1 | 20-01-2005 |
| WO 2005032703 | A | 14-04-2005 | AT 362395 T | 15-06-2007 |
| | | | AU 2004277750 A1 | 14-04-2005 |
| | | | BR PI0414982 A | 21-11-2006 |
| | | | CA 2539303 A1 | 14-04-2005 |
| | | | CN 1863588 A | 15-11-2006 |
| | | | DE 602004006523 T2 | 31-01-2008 |
| | | | DK 1667790 T3 | 24-09-2007 |
| | | | EP 1667790 A1 | 14-06-2006 |
| | | | ES 2286674 T3 | 01-12-2007 |
| | | | JP 2007507339 T | 29-03-2007 |
| | | | KR 20060104989 A | 09-10-2006 |
| | | | MX PA06003599 A | 20-06-2006 |
| | | | US 2007071825 A1 | 29-03-2007 |

EPO FORM P0459

For more details about this annex : see Official Journal of the European Patent Office, No. 12/82

68

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 355578 A, Paltz, R. M.; Patton, J. S.; Foster, L.; Mohammed, E.
- EP 1652836 A
- IS 200602146
- US 6475493 B
- US 20050020539 A1
- WO 2007021970 A, Tan
- US 20050013866 A1
- WO 2006023207 A, Heller, Phillip F.
- US 20050281871 A1

### Non-patent literature cited in the description

- **ALPAR, H. J. ; SOMAVARAPU, S. ; ATUAH, K. N. ; BRAMWELL, V. W.** *Adv. Drug Deliv. Rev.,* 2005, vol. 57, 411-430
- **XIA et al.** *Journal of Medical Virology,* 2006, vol. 79 (1), 74-83
- Delivery systems and adjuvants for oral vaccines. *Opinion on Drug Delivery,* 2006, vol. 3 (6), 747-762
- **ROSS et al.** *Current Drug Delivery,* 2005, vol. 2 (3), 277-287
- **ROSS, BENJAMIN et al.** *Current Drug Delivery,* 2005, vol. 2 (3), 277-28
- **ARBI et al.** *Thrombosis Journal,* 2006
- **XIMELAGATRAN. CHOUDHURY et al.** *Drugs of Today,* 2006, vol. 42 (1), 3-19
- **GANGWAR et al.** Prodrug strategies to enhance the intestinal absorption of peptides. *Drug Discovery Today,* 1997, vol. 2 (4), 148-155
- **KING et al.** Pharmacokinetic enhancement of protease inhibitor therapy. *Clinical Pharmacokinetics,* 2004, vol. 43 (5), 291-310
- Oral delivery of macromolecules using intestinal patches: applications for insulin delivery. *Journal of Controlled Release,* 2004, vol. 98 (1), 37-45
- **EAIMTRAKARN et al.** Retention and transit of intestinal mucoadhesive films in the rat small intestine. *International Journal of Pharmaceutics,* 2001, vol. 224, 61-67
- **LE CORRE, P. ; RYTTING, J. H. ; GAJAN, V. ; CHEVANNE, F. ; LE VERGE, R.** *J. Microencapsulation,* 1997, vol. 14, 243
- **BLANCO, M. D. ; BERNARDO, M. V. ; GÓMEZ, C. ; MUÑIZ, E. ; TEIJÓN, J. M.** *Biomaterials,* 1999, vol. 20, 1919
- **ESTEBE, J. P. ; LE CORRE, P ; CHEVANNE, F. ; MALLJDANT, Y. ; LE VERGE, R.** *Anesth. Analg.,* 1995, vol. 81, 99
- **SÁEZ et al.** Liberación Controlada de Fármacos. *Revista Iberoamericana de Polímeros,* 2001, vol. 5 (1
- **BENOIT, M. et al.** *Biopharmaceutics and Pharmaceutical Technology,* 09 May 1995, 431-2
- **ALBRECHT, K. et al.** Preparation of thiomer microparticles and in vitro evaluation of parameters influencing their mucoadhesive properties. *Drug Development and Industrial Pharmacy,* 2006, vol. 32 (10), 1149-1157
- **DAMGE, C. et al.** Intestinal absorption of PLGA microspheres in the rat. *Journal of Anatomy,* 1996, vol. 189 (3), 491-501
- **PREGO, C. et al.** Transmucosal macromolecular drug delivery. *Department Journal of Controlled Release,* 2005, vol. 101 (1-3), 151-162
- **DESAI et al.** Gastrointestinal uptake of biodegradable microparticles: effect of particle size. *Pharmaceutical Research,* 1996, vol. 13 (12), 1838-1845
- **BHAVSAR et al.** Polymeric nano- and microparticle technologies for oral gene delivery. *Expert Opinion on Drug Delivery,* 2007, vol. 4 (3), 197-213
- **HANG et al.** *International Journal of Pharmaceutics,* 2006, vol. 327 (1-2), 153-159
- **ZHANG et al.** Investigation of lectin-modified insulin liposomes as carriers for oral administration. *International Journal of Pharmaceutics,* 2005, vol. 294 (1-2), 247-259
- **TORRADO, J. J. ; CADÓRNIGA, R.** *Vectorización, CIF,* 1989, vol. 8, 242
- **TORRADO, J. J. ; CADÓRNIGA, R.** *Farm. Clin.,* 1989, vol. 6, 724
- **ROLLOT, J. M. ; COUVREUR, P. ; ROBLOT-TREUPEL, L. ; PUISIEUX, F.** *J. Pharm. Sci,* 1986, vol. 75, 361-364
- Oral bioavailability of a low molecular weight heparin using a polymeric delivery system. *Journal of Controlled Release,* 2006, vol. 113, 38

- Microencapsulation of Low Molecular Weight heparin into Polymeric Particles Designed with Biodegradable and Nonbiodegradable Polycationic Polymers. *Drug Delivery,* 2003, vol. 10, 1
- **WOOD, R. W. ; LI, V. H. K. ; KREUTER, J. ; ROBINSON, J. R.** *Int. J. Pharm.,* 1985, vol. 23, 175
- **YOSHIKAWA, H. ; NAKAO, Y. ; TAKADA, K. ; MURANISHI, S. ; WADA, R. T. ; TABATA, Y. ; HYON, S. H. ; IKADA, Y.** *Chem. Pharm. Bull.,* 1989, vol. 37, 802
- **SÁNCHEZ, A. ; VILA-JATO, J. L. ; ALONSO, M. J.** *Int. J. Pharm.,* 1993, vol. 99, 263
- **SÁNCHEZ, A. ; VILA-JATO, J. L. ; ALONSO, M. J.** *Int. J. Pharm.,* 1993, vol. 99, 263J
- **DUBEMET, C. ; BENOIT, J. P. ; COUARRAZE, G. ; DUCHÈNE, D.** *Int. J. Pharm.,* 1987, vol. 35, 145
- **ELDRIGE, J. H. ; STAAS, J. K. ; MEULBROCK, J. A. ; MCGHEE, J. R. ; TICE, T. R. ; GILLEY, R. M.** *Mol. Immunol.,* 1991, vol. 28, 287
- **REIS et al.** Nanoencapsulation. II. Biomedical applications and current status of peptide and protein nanoparticulate delivery systems. *Nanomedicine,* 2006, vol. 2 (2), 53-65
- **BHAVSAR et al.** Polymeric nano-and microparticle technologies for oral gene delivery. *Expert Opinion on Drug Delivery,* 2007, vol. 4 (3), 197-213
- **RYDELL et al.** Starch microparticles as vaccine adjuvant. *Expert Opinion on Drug Delivery,* 2005, vol. 2 (5), 807-828
- **LAMEIRO et al.** Encapsulation of adenoviral vectors into chitosan-bile salt microparticles for mucosal vaccination. *Journal of Biotechnology,* 2006, vol. 126 (2), 152-162
- **C. PENICHE et al.** Chitosan: An Atractive biocompatible polymer for macroencasulation. *Macromolecules Bioscience,* 2003, vol. 3, 511-520
- **ACOSTA et al.** Tramadol release from delivery system based on alginate-chitosan microcapsules. *Macromolecules Bioscience,* 2003, vol. 3, 546-551
- **SUNKARA et al.** Drug delivery applications of supercritical fluid technology. *Drug Delivery Technology,* vol. 2 (1), 44, 46-50
- **TAKEUCHI et al.** Mucoadhesive nanoparticulate systems for peptide drug delivery. *Advanced Drug Delivery Reviews,* 2001, vol. 47, 39-54
- **TAKEUCHI et al.** Enteral absorption of insulin in rats from mucoadhesive chitosan-coated liposomes. *Pharmaceutical Research,* 1996, vol. 13, 896-901
- **MORISHITA et al.** Mucosal insulin delivery systems based on complexation polymer hydrogels: effect of particle size on insulin enteral absorption. *Journal of Controlled Release,* 2004, vol. 97, 115-124
- Colloidal delivery systems-Opportunities and challenges. **DAVIS, S. S. ; ILLUM, L.** Site-Specific Drug Delivery. John Wiley & Sons Ltd, 1986, 93-110
- **DUNCAN, R. ; KOPEKOVA-REJMANOVA, P. ; STROHALM, J. ; HUME, I. ; CABLE, H. C. ; POHL, J. ; LLOYD, B. ; KOPECEK, J.** *Br. J.Cancer,* 1987, vol. 55, 165-174
- **ENDO, N. ; UMEMOTO, N. ; KATO, Y. ; TAKEDA, Y. ; HARA, T.** *J. Immunol. Methods,* 1987, vol. 104, 253-258
- **ZUNINO, F. ; PRATESI, G. ; MICHELONI, A.** *J. Control. Rel.,* 1989, vol. 10, 65-73
- **CHU, B.** *Langmuir,* 1995, vol. 11, 414-421
- **HOMAR et al.** *J Microencap,* 2007, vol. 24 (7), 621-633
- **VARDE et al.** Microspheres for controlled release drug delivery. *Expert Opinion on Biological Therapy,* 2004, vol. 4 (1), 35-51
- **BILATI et al.** Strategic approaches for overcoming peptide and protein instability within biodegradable nano- and microparticles. *European Journal of Pharmaceutics and Biopharmaceutics,* 2005, vol. 59 (3), 375-388
- **FLORENCE, ALEXANDER T.** Issues in oral nanoparticle drug carrier uptake and targeting. *Journal of Drug Targeting,* 2004, vol. 12 (2), 65-70
- **MORRIS et al.** Potential of polymer microencapsulation technology for vaccine innovation. *Vaccine,* 1994, vol. 12 (1), 5-11
- **LIANG et al.** Particulate systems as adjuvants and carriers for peptide and protein antigens. *Current Drug Delivery,* 2006, vol. 3 (4), 379-388
- **KLEINEBUDDE, PETER.** Roll compaction/dry granulation: pharmaceutical applications. *European Journal of Pharmaceutics and Biopharmaceutics,* 2004, vol. 58 (2), 317-326
- **KARASULU et al.** Theophylline granule formulation prepared by the wet granulation method: comparison of in vitro dissolution profiles and estimation of in vivo plasma concentrations. *European Journal of Drug Metabolism and Pharmacokinetics,* 2007, vol. 31 (4), 291-298
- **RAJNIAK, P et al.** Experimental study of wet granulation in fluidized bed: Impact of the binder properties on the granule morphology. *International Journal of Pharmaceutics,* 2007, vol. 334 (1-2), 92-102
- **THE-MAHROUK et al.** Preparation and evaluation of sustained release indomethacin nonpareil seeds. *Drug Development and Industrial Pharmacy,* 1993, vol. 19 (15), 1903-16
- **NEWTON et al.** The preparation of pellets containing a surfactant or a mixture of mono- and di-glycerides by extrusion/spheronization. *European Journal of Pharmaceutical Sciences,* 2007, vol. 30 (3-4), 333-342
- **WU et al.** Characterization of 5-Fluorouracil Release from Hydroxypropylmethylcellulose Compression-Coated Tablets. *Pharmaceutical Development and Technology,* 2007, vol. 12 (2), 203-210

- **SILVA, OR. et al.** In vitro dissolution studies of sodium diclofenac coated granules with eudragit L-30D-55 by fluidized-bed system. *Drug Development and Industrial Pharmacy,* 2006, vol. 32 (6), 661-667
- Low- and ultra-low-molecular-weight heparins. *Best Pract Res Clin Haemal.,* 2004, vol. 17, 77-87
- Prevention of venous thromboembolism. Agnelli and Sonaglia. *Tromb Res.,* 2000, vol. 97, 49-62
- Treatment of venous thromboembolism. *Ageno. Tromb Res.,* 2000, vol. 97, 63-72
- **HETZEL et al.** Heparins: all a nephrologist should know. *Nephrol Dial Transplant.,* 2005, vol. 20, 2036-42
- **ANDERSSON et al.** Molecular weight dependency of the heparin potentiated inhibition of thrombin and activated factor X. Effect of heparin neutralization in plasma. *Thromb Res.,* 1979, vol. 15, 531-41
- **HIRSH ; RASCHKE.** Heparin and Low-Molecular-Weight Heparin: The Seventh ACCP Conference on Antithrombotic and Thrombolytic Therapy. *Chest.,* 2004, vol. 126, 188S-203S
- **HIRSH ; RASCHKE.** Heparin and Low-Molecular-Weight Heparin: The Seventh ACCP Conference on Antithrombotic and Thrombolytic Therapy. *Chest.,* 2004, vol. 126, 188S-203S
- **MONEY ; GORKA.** Development of oral heparin therapy for prophylaxis and treatment of deep venous thrombosis. *Caridovasc Surg.,* 2001, vol. 9, 211-8
- **AGNELLI ; SONAGLIA.** Prevention of venous thromboembolism. *Tromb Res.,* 2000, vol. 97, 49-62
- **UBRICH et al.** Digestive absorption of heparin with alternative formulations. *S.T.P. Pharma Sciences.,* 2002, vol. 12, 147-55
- **TEIEN ; LIE.** Evaluation of an amidolytic heparin assay method: increased sensitivity by adding purified antithrombin III. *Thromb Res.,* 1977, vol. 10, 399-410
- **CHEN DR et al.** Polycaprolactone microparticles and their biodegradation. *Polymer degradation and stability,* 2000, vol. 52, 117-126